# EUROPEAN PATENT APPLICATION

(11) **EP 3 530 283 A1**
(43) Date of publication of application: **28.08.2019**
(21) Application number: 19150395.2
(22) Date of filing: 05.06.2013
(51) Int. Cl.: A61K 38/20, A61K 39/00, A61K 39/21, A61K 39/12, A61P 31/18

(54) **VACCINATION WITH INTERLEUKIN-4 ANTAGONISTS**

(30) Priority: 05.06.2012 AU 2012902345
(62) Divisional of application: 13800233.2
(71) Applicant: The Australian National University, Acton, ACT 0200 (AU)
(72) Inventor: Jackson, Ronald James, McKellar Australian Capital Territory 2617 (AU); Ranasinghe, Charani, Aranda Australian Capital Territory 2614 (AU)
(74) Representative: Potter Clarkson

(57) **Abstract**

The invention relates to methods for inducing an antigen-specific immune response, methods for increasing the avidity of immune cells for an antigen, methods for increasing the number of immune cells specific for an antigen, methods of preventing or treating infection and methods of vaccinating, the methods comprising administering an interleukin-4 receptor (LL-4R) antagonist in combination with an antigen, in particular HIV-1 antigens selected from a gag, pol, or env.

## Description

### INCORPORATION BY CROSS-REFERENCE

The present application claims priority from Australian provisional patent application number 2012902345 filed on 5 June 2012, the entire contents of which are incorporated herein by cross-reference.

### TECHNICAL FIELD

The present invention relates generally to the field of immunology. More specifically, the present invention relates to compositions and methods for enhancing antigen-specific immune responses. The compositions and methods find particular application in the prevention and/or treatment of various diseases including, but not limited to, infectious diseases.

### BACKGROUND

It is widely felt that potent antiviral mucosal CD8⁺ cytotoxic T lymphocytes (CTL) should be a major component of an immune response induced by viral vaccines. In particular, the importance of local antiviral immune responses at mucosal surfaces is becoming increasingly clear. It is also becoming increasingly evident that not only the magnitude but also the 'quality' or avidity of the antiviral T-cell responses may be important in protecting against viral infection, particularly those proven to be resistant to existing immunisation strategies. The quality of the T-cell response may be reflected in the functional avidity of T cells towards the MHC-peptide complex on target cells. High-avidity CTL can recognise low concentrations of antigen, while low-avidity CTL are inefficient in effector function at low concentrations of antigen. It has been established that high-avidity CTL have increased functional capacity to clear infection compared with low-avidity T cells. However, the relative ratio of high- and low-avidity CTL has been observed to change over the course of infection. Nonetheless, it well recognised that high avidity CTL have a greater ability to clear an infection than low avidity T cells.

For example, in the case of human immunodeficiency virus (HIV) local antiviral immune responses in the genital and rectal tissues where HIV is usually first encountered are vital. Local immune responses in the gastro-intestinal tract are also important given that it is a major site of HIV replication. The presence of high-avidity antiviral T cells at sites of initial HIV exposure (mucosa) offers the potential for reducing mucosal CD4⁺ T-cell depletion and local control of HIV infection. Given their demonstrated capacity to recognise target cells expressing very low levels of viral antigen (e.g. early after infection of a cell), and their more rapid initiation of target cell lysis at low concentrations of target antigen, vaccine strategies that elicit high-avidity CTL are potentially likely to offer greater protection against infection.

A need exists for antiviral vaccines capable of eliciting high-avidity CTL. In particular, there is a need for antiviral vaccines capable of eliciting high-avidity CTL at mucosal surfaces which are the initial point of infection for many viruses.

### SUMMARY OF THE INVENTION

The present inventors have discovered that both the magnitude and avidity of antigen-specific T lymphocytes induced by antiviral vaccines can be increased by transiently reducing the availability of interleukin-4 (IL-4) and/or transiently inhibiting IL-4 function.

In a first aspect, the present invention provides a method for inducing an antigen-specific immune response in a subject, the method comprising administering the antigen to the subject in combination with an interleukin-4 receptor (IL-4R) antagonist.

In a second aspect, the present invention provides a method for increasing the avidity of immune cells for an antigen in a subject, the method comprising administering the antigen to the subject in combination with an interleukin-4 receptor (IL-4R) antagonist.

In a third aspect, the present invention provides a method for increasing the number of immune cells specific for an antigen in a subject, the method comprising administering the antigen to the subject in combination with an interleukin-4 receptor (IL-4R) antagonist.

In one embodiment of the above aspects, the method comprises administering a priming dose and a booster dose to the subject, each said dose comprising administering the antigen to the subject in combination with the interleukin-4 receptor (IL-4R) antagonist.

In one embodiment of the above aspects, the method comprises administering a priming dose to the subject, wherein the priming dose comprises the antigen and an IL-4R antagonist.

In one embodiment of the above aspects, the method comprises administering a booster dose to the subject, wherein the booster dose comprises the antigen and an IL-4R antagonist.

In one embodiment of the above aspects, the antigen and IL-4R antagonist of the booster dose may be administered sequentially or simultaneously.

In one embodiment of the above aspects, administering the primer dose comprises administering a polynucleotide encoding the antigen, a polynucleotide encoding the IL-4R antagonist, or both.

In one embodiment of the above aspects, administering the booster dose comprises administering a polynucleotide encoding the antigen, a polynucleotide encoding the IL-4R antagonist, or both.

In one embodiment of the above aspects, any one or more of the polynucleotides in the primer dose may be a component of a recombinant virus.

In one embodiment of the above aspects, any one or more of the polynucleotides in the booster dose may be a component of a recombinant virus.

In one embodiment of the above aspects, the recombinant virus of the primer dose is a poxvirus.

In one embodiment of the above aspects, the recombinant virus of the booster dose is a poxvirus.

In one embodiment of the above aspects, the recombinant virus of the primer dose is a poxvirus selected from the group consisting of a vaccinia virus, an attenuated vaccinia virus (e.g. NYVAC), a fowlpox virus, a canarypox virus, and a modified vaccinia ankara (MVA) virus.

In one embodiment of the above aspects, the recombinant virus of the booster dose is a poxvirus selected from the group consisting of a vaccinia virus, an attenuated vaccinia virus (e.g. NYVAC), a fowlpox virus, a canarypox virus, and a modified vaccinia ankara (MVA) virus.

In one embodiment of the above aspects, the IL-4R antagonist of the primer dose is an interleukin-4 receptor alpha chain (IL-4Rα) antagonist capable of binding to IL-4Rα and preventing IL-4R signalling.

In one embodiment of the above aspects, the IL-4R antagonist of the booster dose is an interleukin-4 receptor alpha chain (IL-4Rα) antagonist capable of binding to IL-4Rα and preventing IL-4R signalling.

In one embodiment of the above aspects, the IL-4R antagonist of the primer dose is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.

In one embodiment of the above aspects, the IL-4R antagonist of the booster dose is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.

In one embodiment of the above aspects, the IL-4R antagonist of the primer dose is a human interleukin-4 molecule comprising a mutation selected from: R121D, Y124D, a deletion of a fragment encoded by exon 2 of an interleukin-4 gene, or any combination thereof.

In one embodiment of the above aspects, the IL-4R antagonist of the booster dose is a human interleukin-4 molecule comprising a mutation selected from: R121D, Y124D, a deletion of a fragment encoded by exon 2 of an interleukin-4 gene, or any combination thereof.

In one embodiment of the above aspects, the administering comprises administering a polynucleotide encoding the antigen, a polynucleotide encoding the interleukin-4 receptor antagonist, or both.

In one embodiment of the above aspects, any one or more of said polynucleotides is a component of a recombinant virus.

In one embodiment of the above aspects, the recombinant virus is a poxvirus.

In one embodiment of the above aspects, the recombinant virus is a poxvirus selected from the group consisting of a vaccinia virus, an attenuated vaccinia virus (e.g. NYVAC), a fowlpox virus, a canarypox virus, and a modified vaccinia ankara virus.

In one embodiment of the above aspects, the recombinant virus transiently expresses the interleukin-4 receptor (IL-4R) antagonist in the subject.

In one embodiment of the above aspects, the administering of the antigen to the subject in combination with an interleukin-4 receptor (IL-4R) antagonist decreases the number of CD103+, b220+, CD8α+ cells in the subject and/or increases CD11b+, CD103-,b220-, CD8α - cells in the subject. The CD103+, b220+, CD8α+ cells and/or CD11b+, CD103-, b220-, CD8α - cells may be specific for the antigen. The antigen may be an HIV antigen (e.g. HIV gag, pol and/or env antigen).

In a fourth aspect, the present invention provides the use of an antigen and an interleukin-4 receptor (IL-4R) antagonist in the manufacture of a medicament for inducing a specific immune response against the antigen in a subject.

In a fifth aspect, the present invention provides the use of an antigen and an interleukin-4 receptor (IL-4R) antagonist in the manufacture of a medicament for increasing the avidity of immune cells specific for the antigen in a subject.

In a sixth aspect, the present invention provides the use of an antigen and an interleukin-4 receptor (IL-4R) antagonist in the manufacture of a medicament for increasing the number of immune cells specific for the antigen in a subject.

In a seventh aspect, the present invention provides an antigen and an interleukin-4 receptor (IL-4R) antagonist for use in inducing a specific immune response against the antigen in a subject.

In an eighth aspect, the present invention provides an antigen and an interleukin-4 receptor (IL-4R) antagonist for use in increasing the avidity of immune cells specific for the antigen in a subject.

In a ninth aspect, the present invention provides an antigen and an interleukin-4 receptor (IL-4R) antagonist for use in increasing the number of immune cells specific for the antigen in a subject.

In one embodiment of the fourth, fifth, sixth, seventh, eighth, and ninth aspects, the antigen is provided as a polynucleotide encoding the antigen.

In one embodiment of the fourth, fifth, sixth, seventh, eighth, and ninth aspects, the interleukin-4 receptor antagonist is provided a polynucleotide encoding the interleukin-4 receptor antagonist.

In one embodiment of the fourth, fifth, sixth, seventh, eighth, and ninth aspects, any one or more of said polynucleotides is a component of a recombinant virus.

In one embodiment of the fourth, fifth, sixth, seventh, eighth, and ninth aspects, the recombinant virus is a poxvirus.

In one embodiment of the fourth, fifth, sixth, seventh, eighth, and ninth aspects, the recombinant virus is a poxvirus selected from the group consisting of a vaccinia virus, an attenuated vaccinia virus (e.g. NYVAC), a fowlpox virus, a canarypox virus, and a modified vaccinia ankara virus.

In one embodiment of the fourth, fifth, sixth, seventh, eighth, and ninth aspects, the recombinant virus transiently expresses the interleukin-4 receptor (IL-4R) antagonist in the subject.

In one embodiment of the fourth, fifth, and sixth aspects, the medicament is a vaccine.

In one embodiment of the fourth, fifth, and sixth aspects, the medicament is a vaccine comprising first and second components, wherein the first component comprises the interleukin-4 receptor (IL-4R) antagonist and the second component comprises the antigen, and wherein the first and second components are for, or are formulated for, simultaneous or sequential administration to the subject.

In one embodiment of the seventh, eighth, and ninth aspects aspects, the antigen and interleukin-4 receptor (IL-4R) antagonist are components of a vaccine.

In one embodiment of the fourth, fifth, sixth, seventh, eighth and ninth aspects, the vaccine is for administration to the subject, or formulated for administration to the subject, as a primer dose, a booster dose, or both.

In one embodiment of the fourth, fifth, sixth, seventh, eight and ninth aspects, the vaccine is capable of decreasing the number of CD103+, b220+, CD8α+ cells in the subject and/or increasing CD11b+, CD103-,b220-, CD8α - cells in the subject, upon administration to the subject. The CD103+, b220+, CD8α+ cells and/or CD11b+, CD103-, b220-, CD8α - cells may be specific for the antigen. The antigen may be an HIV antigen (e.g. HIV gag, pol and/or env antigen).

In one embodiment of the first, second, third, fifth, sixth, eighth and ninth aspects, the immune cell is a T-lymphocyte.

In one embodiment the first, second, third, fifth, sixth, eighth and ninth aspects, the immune cell is a CD8⁺ T-lymphocyte.

In one embodiment the first, second, third, fifth, sixth, eighth and ninth aspects, the immune cell is a cytotoxic CD8⁺ T-lymphocyte.

In one embodiment of the above aspects, the antigen is a viral antigen.

In one embodiment of the above aspects, the antigen is a human immunodeficiency virus antigen.

In one embodiment of the above aspects, the antigen is a human immunodeficiency virus antigen selected from a gp120 env, gp140 env, gp160 env, p18, gag, pol, vif, vpr, vpu, tat, rev, and nef gene product, and combinations thereof.

In one embodiment of the above aspects, the antigen is a human immunodeficiency virus antigen selected from a gag, pol, or env gene product, or any combination thereof.

In one embodiment of the above aspects, the antigen is a human immunodeficiency virus antigen selected from a combined gag/pol, or a combined gag/pol/env gene product.

In one embodiment of the above aspects, the antigen and interleukin-4 receptor antagonist are administered to the subject sequentially.

In one embodiment of the above aspects, the antigen and interleukin-4 receptor antagonist are administered to the subject simultaneously.

In one embodiment of the above aspects, the antigen and interleukin-4 receptor antagonist are administered to the subject as a priming dose.

In one embodiment of the above aspects, the IL-4R antagonist is an interleukin-4 receptor alpha chain (IL-4Rα) antagonist capable of binding to IL-4Rα and preventing IL-4R signalling.

In one embodiment of the above aspects, the IL-4R antagonist is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.

In a tenth aspect, the present invention provides a composition comprising:
(i) an antigen and/or a polynucleotide encoding the antigen; and
(ii) an interleukin-4 receptor (IL-4R) antagonist and/or a polynucleotide encoding the IL-4R antagonist.

In an eleventh aspect, the present invention provides the use of an antigen and an interleukin-4 receptor (IL-4R) antagonist in the manufacture of a medicament for preventing or treating an infection in a subject.

In a twelfth aspect, the present invention provides the use of an antigen and an interleukin-4 receptor (IL-4R) antagonist in the manufacture of a medicament for vaccinating a subject against an infection.

In a thirteenth aspect, the present invention provides an antigen and an interleukin-4 receptor (IL-4R) antagonist for use in preventing or treating an infection in a subject.

In a fourteenth aspect, the present invention provides the use of an antigen and an interleukin-4 receptor (IL-4R) antagonist for use in vaccinating a subject against an infection.

In one embodiment of the eleventh aspect the medicament is a vaccine.

In one embodiment of the thirteenth aspect the antigen and interleukin-4 receptor (IL-4R) antagonist are components of a vaccine.

In one embodiment of the eleventh and thirteenth aspects, the vaccine comprises first and second components, wherein the first component comprises the interleukin-4 receptor (IL-4R) antagonist and the second component comprises the antigen, and wherein the first and second components are for, or are formulated for, simultaneous or sequential administration to the subject.

In one embodiment of the eleventh and thirteenth aspects, the vaccine is for administration to a subject, or formulated for administration to a subject, as a primer dose, a booster dose, or both.

In one embodiment of the twelfth aspect, the medicament is for administration to the subject, or formulated for administration to the subject, as a primer dose, a booster dose, or both.

In one embodiment of the fourteenth aspect, the antigen and interleukin-4 receptor (IL-4R) antagonist is for administration to the subject, or formulated for administration to the subject, as a primer dose, a booster dose, or both.

In one embodiment of the eleventh and thirteenth aspects, the vaccine is capable of decreasing the number of CD103+, b220+, CD8α+ cells in the subject and/or increasing CD11b+, CD103-,b220-, CD8α - cells in the subject, upon administration to the subject. The CD103+, b220+, CD8α+ cells and/or CD11b+, CD103-, b220-, CD8α - cells may be specific for the antigen. The antigen may be an HIV antigen (e.g. HIV gag, pol and/or env antigen).

In one embodiment of the twelfth and fourteenth aspects, said vaccinating is capable of decreasing the number of CD103+, b220+, CD8α+ cells in the subject and/or increasing CD11b+, CD103-, b220-, CD8α - cells in the subject. The CD103+, b220+, CD8α+ cells and/or CD11b+, CD103-, b220-, CD8α - cells may be specific for the antigen. The antigen may be an HIV antigen (e.g. HIV gag, pol and/or env antigen).

In one embodiment of the eleventh, twelfth, thirteenth and fourteenth aspects, the antigen is provided as a polynucleotide encoding the antigen.

In one embodiment of the eleventh, twelfth, thirteenth and fourteenth aspects, the interleukin-4 receptor is provided as a polynucleotide encoding the interleukin-4 receptor antagonist.

In one embodiment of the tenth, eleventh, twelfth, thirteenth and fourteenth aspects, any one or more of said polynucleotides is a component of a recombinant virus.

In one embodiment of the tenth, eleventh, twelfth, thirteenth and fourteenth aspects, the recombinant virus is a poxvirus.

In one embodiment of the tenth, eleventh, twelfth, thirteenth and fourteenth aspects, the recombinant virus is a poxvirus selected from the group consisting of a vaccinia virus, an attenuated vaccinia virus (e.g. NYVAC), a fowlpox virus, a canarypox virus, and a modified vaccinia ankara virus.

In one embodiment of the eleventh, twelfth, thirteenth and fourteenth aspects, the recombinant virus transiently expresses the interleukin-4 receptor (IL-4R) antagonist in the subject.

In one embodiment of the tenth, eleventh, twelfth, thirteenth and fourteenth aspects, the antigen is a viral antigen.

In one embodiment of the tenth, eleventh, twelfth, thirteenth and fourteenth aspects, the antigen is a human immunodeficiency virus antigen selected from a gp120 env, gp140 env, gp 160 env, p 18, gag, pol, vif, vpr, vpu, tat, rev, and nef gene product, and combinations thereof.

In one embodiment of the tenth, eleventh, twelfth, thirteenth and fourteenth aspects, the antigen is a human immunodeficiency virus antigen selected from a gag, pol, or env gene product, or any combination thereof.

In one embodiment of the tenth, eleventh, twelfth, thirteenth and fourteenth aspects, the antigen is a human immunodeficiency virus antigen selected from a combined gag/pol, or a combined gag/pol/env gene product.

In one embodiment of the tenth, eleventh, twelfth, thirteenth and fourteenth aspects, the IL-4R antagonist is an interleukin-4 receptor alpha chain (IL-4Rα) antagonist capable of binding to IL-Rα and preventing IL-4R signalling.

In one embodiment of the tenth, eleventh, twelfth, thirteenth and fourteenth aspects, the IL-4R antagonist is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.

In one embodiment of the tenth, eleventh, twelfth, thirteenth and fourteenth aspects, the mutation is selected from: R121D, Y124D, a deletion of a fragment encoded by exon 2 of an interleukin-4 gene, or any combination thereof.

In a fifteenth aspect, the present invention provides a method for preventing or treating an infection in a subject, the method comprising administering to the subject the composition according to the tenth aspect.

In a sixteenth aspect, the present invention provides a method of vaccinating a subject against an infection, the method comprising administering a primer dose and a booster dose of the composition according to the tenth aspect.

In one embodiment of the fifteenth and sixteenth aspects, the infection is human immunodeficiency virus infection and the composition comprises a human immunodeficiency virus antigen selected from a gag, pol, or env gene product, or any combination thereof.

In one embodiment of the eleventh, twelfth, thirteenth, and fourteenth aspects, the infection is human immunodeficiency virus infection and the antigen is a human immunodeficiency virus antigen selected from a gag, pol, or env gene product, or any combination thereof.

### BRIEF DESCRIPTION OF THE FIGURES

Preferred embodiments of the present invention will now be described, by way of example only, with reference to the accompanying figures wherein:

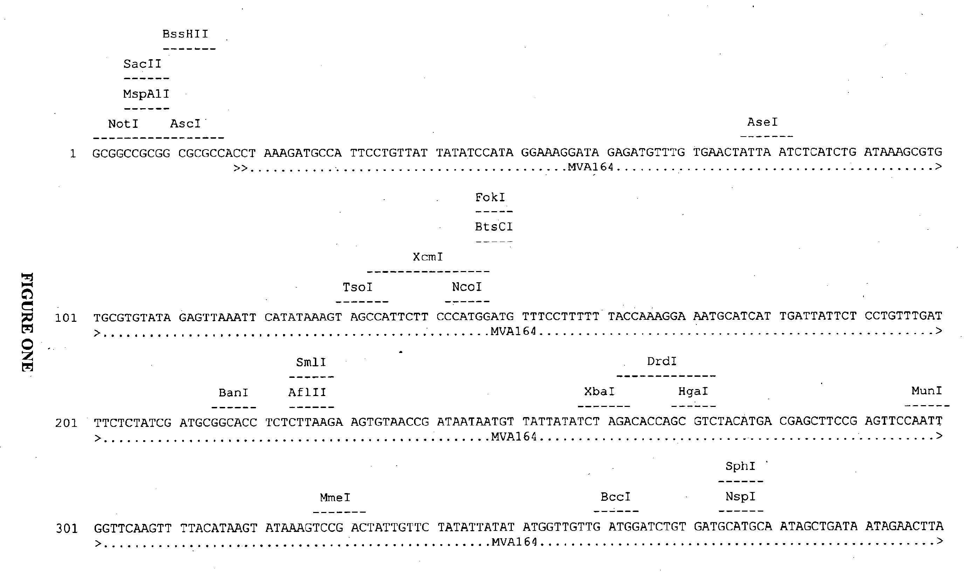

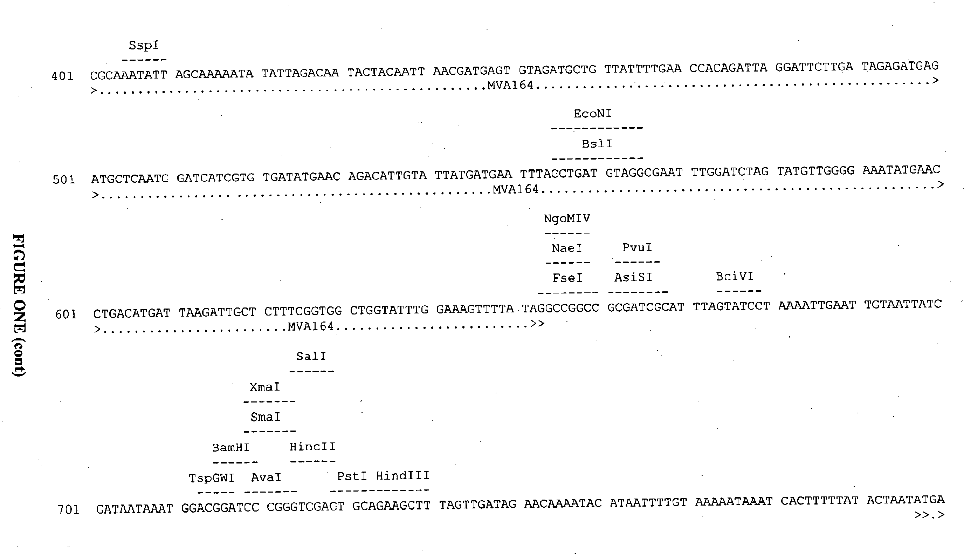

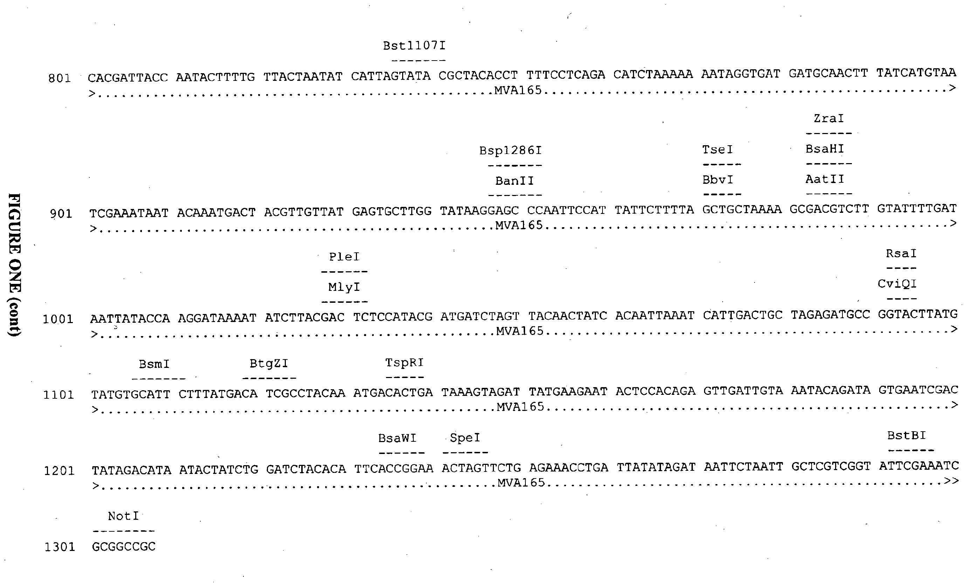

**Figure 1** shows the MVA del-III synthetic vector sequence comprising an intergenic insertion site created between MVA164 and MVA165 including sequences from FseI to HindIII sites, and incorporating a fowlpox virus early/late promoter.

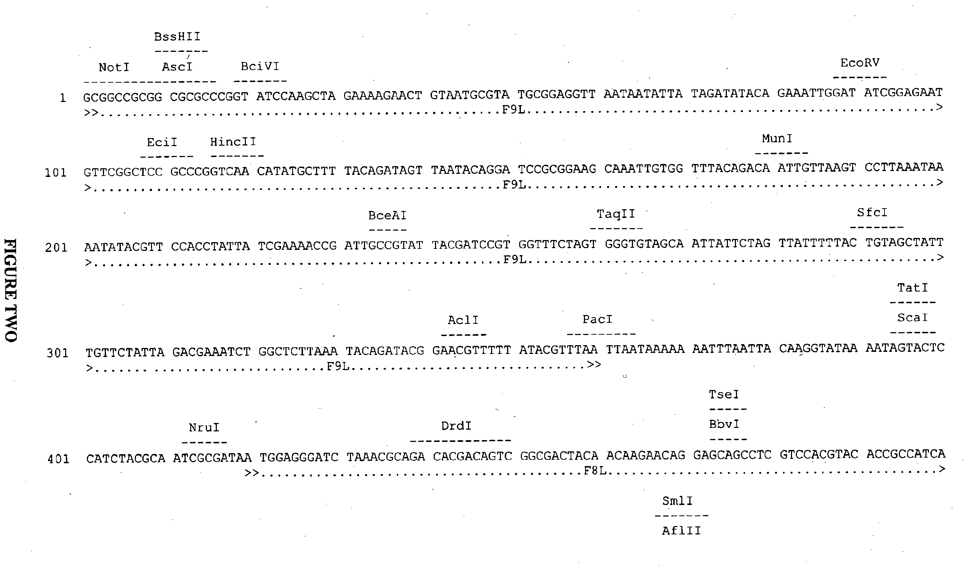

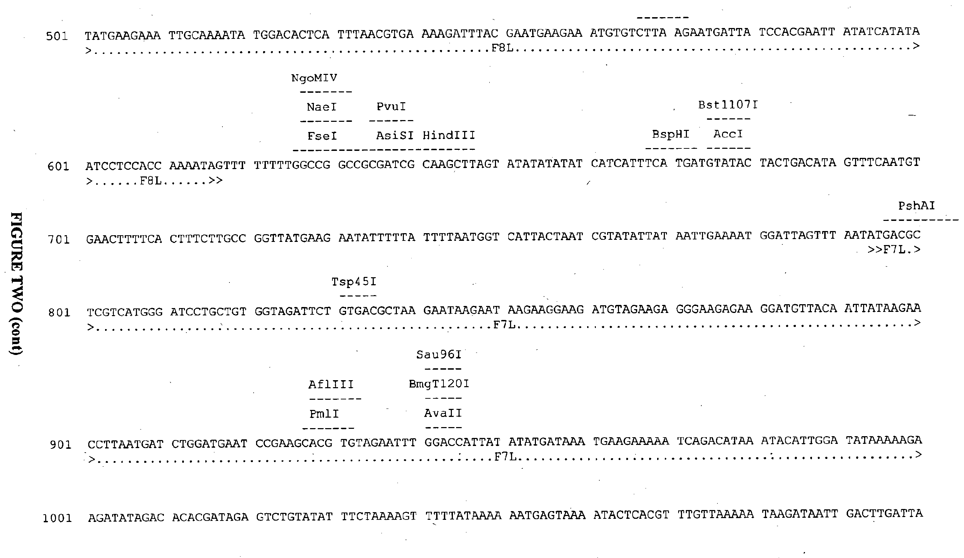

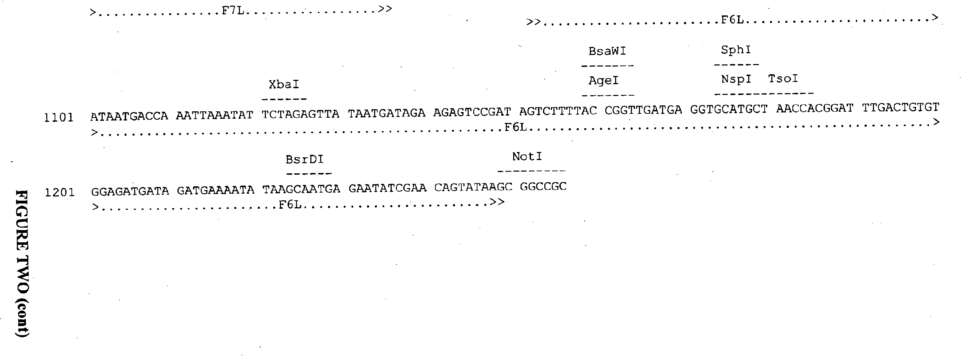

**Figure 2** shows the MVA "HindIII F" insertion site, wherein a multiple cloning site containing FseI to HindIII (including an upstream T5NT early transcription termination signal) has been inserted between ORFs F8L and F7L.

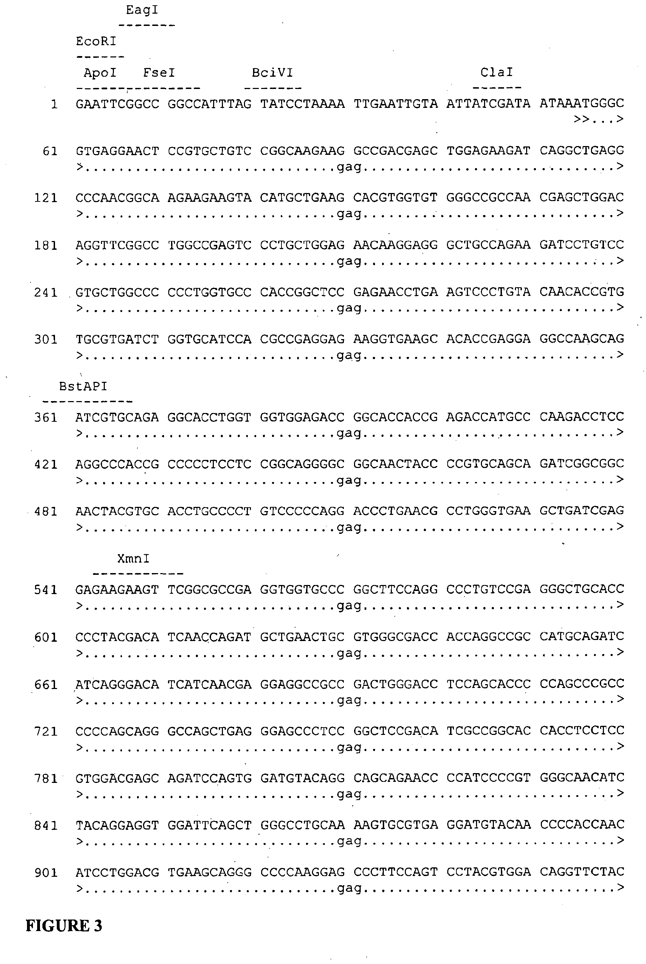

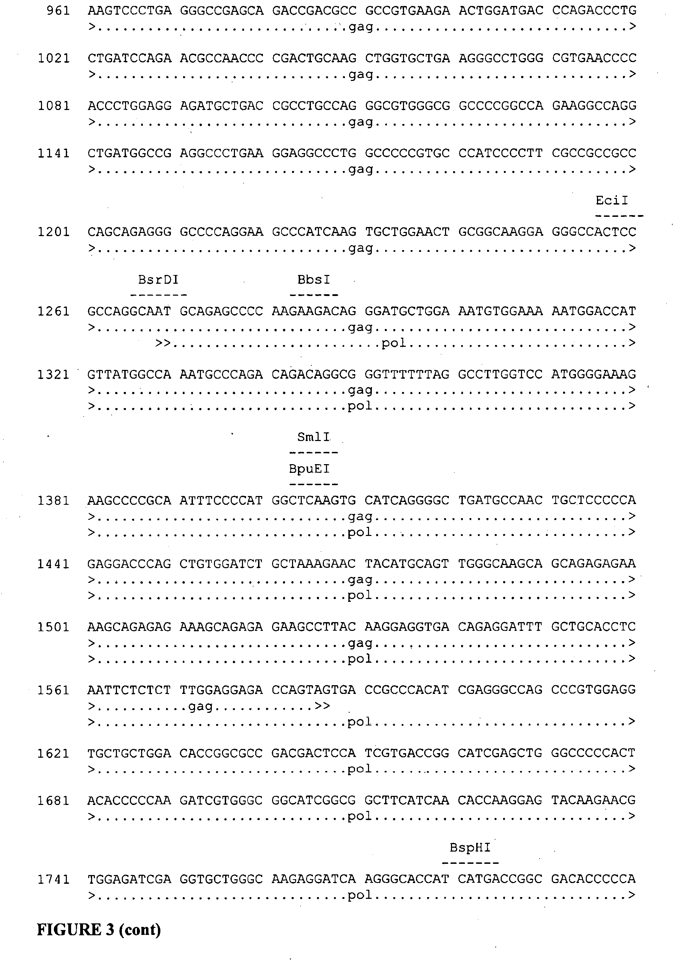

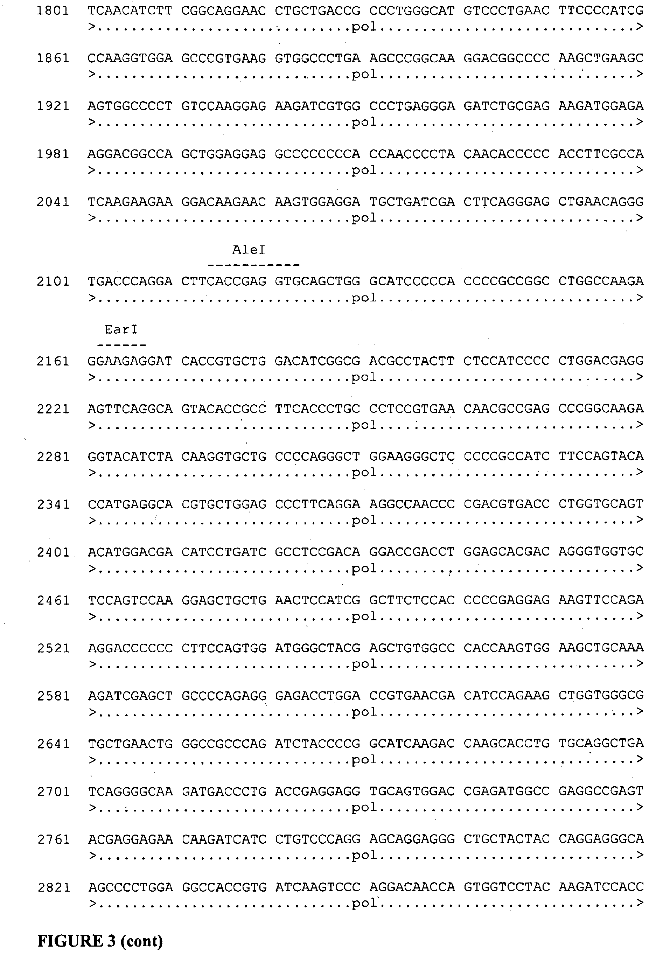

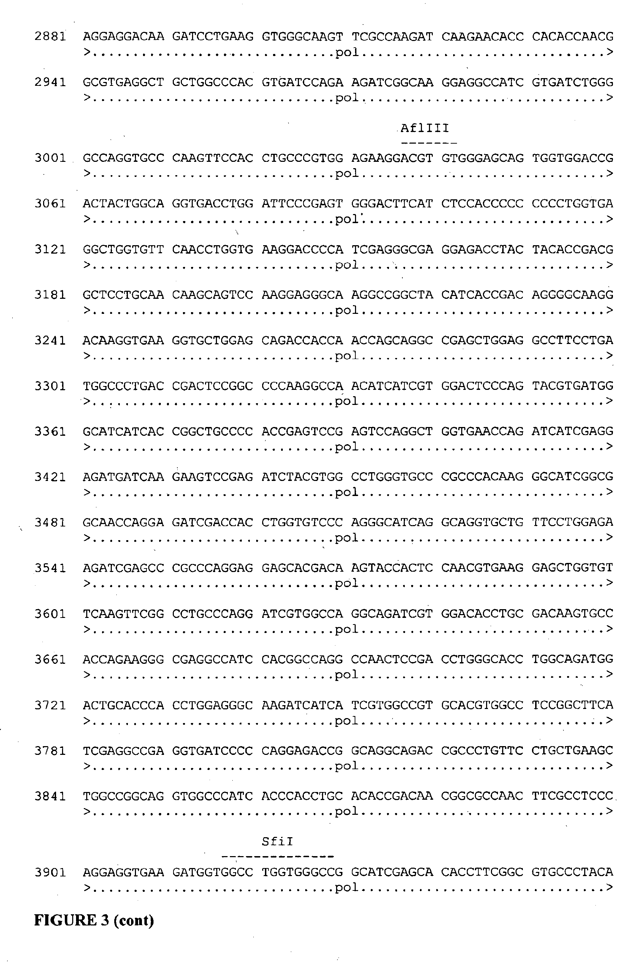

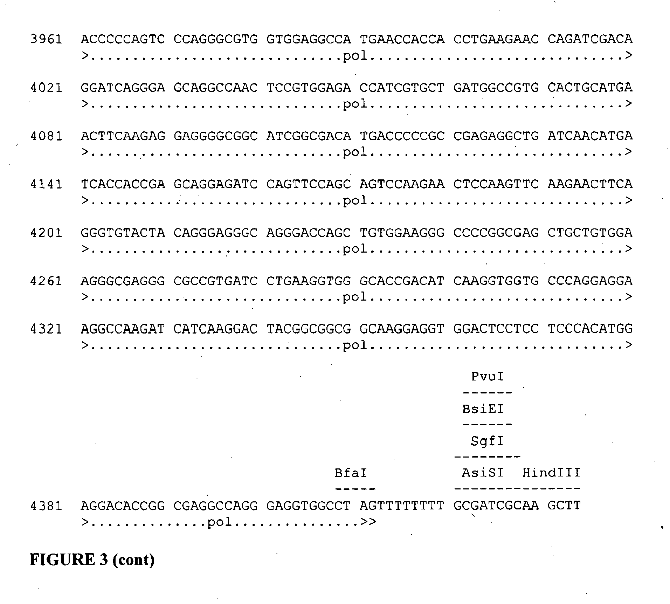

**Figure 3** shows a synthetic SIV mac239 gag/pol sequence optimised for expression in macaque monkeys. The sequence contains a synthetic poxvirus early/late promoter upstream of the genes and an early transcription terminator sequence (T5NT) downstream flanked by restriction endonuclease site to facilitate cloning. The genes are expressed as a fusion protein resulting from a frame-shift during translation in the overlapping region.

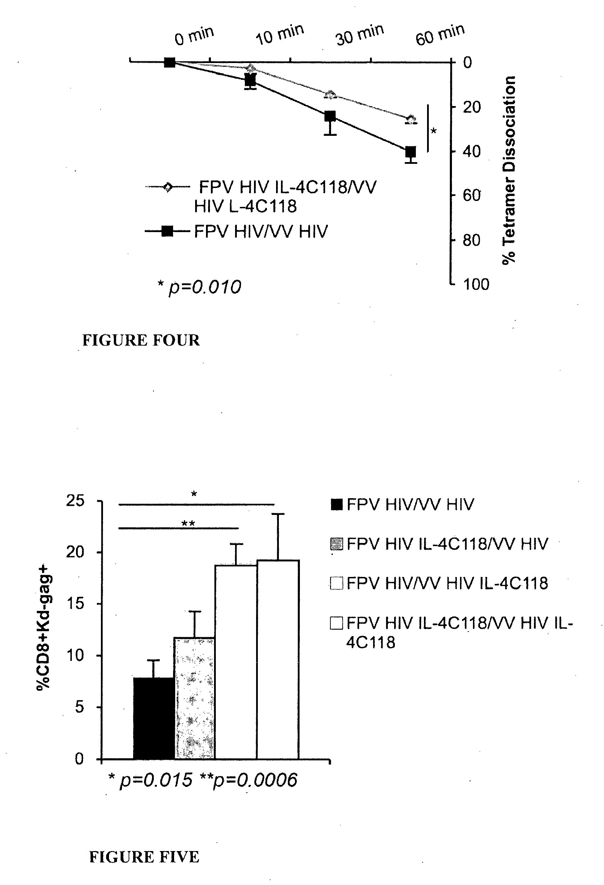

**Figure 4** is a timecourse graph showing HIV KdGag-specific CD8⁺ T-cell avidity following co-vaccination with IL-4 receptor (IL-4R) antagonist (IL-4C118). FPV HIV IL-4C118/VV HIV IL-4C118 = priming vaccination with recombinant fowlpox virus encoding HIV gag/pol antigen and mutant IL-4C118 followed by booster vaccine with recombinant vaccinia virus encoding HIV gag/pol antigen and mutant IL-4C118. FPV HIV/VV HIV = primer vaccination with recombinant fowlpox encoding HIV gag/pol (no IL-4 receptor antagonist) followed by booster vaccination with recombinant vaccinia virus encoding HIV gag/pol (no IL-4 receptor antagonist).
**Figure 5** is a column graph showing HIV KdGag-specific CD8⁺ T-cell avidity and magnitude of HIV KdGag-specific CD8⁺ T-cell responses following co-vaccination with IL-4 receptor antagonist. FPV HIV/VV HIV = priming vaccination with recombinant fowlpox encoding HIV gag/pol (no IL-4 receptor antagonist) followed by booster vaccination with recombinant vaccinia virus encoding HIV gag/pol (no IL-4 receptor antagonist); FPV HIV IL-4C118/VV HIV = priming vaccination with recombinant fowlpox virus encoding HIV gag/pol antigen and mutant IL-4C118 followed by booster vaccine with recombinant vaccinia virus encoding HIV gag/pol (no IL-4 receptor antagonist); FPV HIV/VV HIV IL-4C118 = priming vaccination with recombinant vaccinia virus encoding HIV gag/pol antigen and mutant IL-4C118 followed by booster vaccine with recombinant fowlpox virus encoding HIV gag/pol (no IL-4 receptor antagonist); FPV HIV IL-4C118/VV HIV IL-4C118 = priming vaccination with recombinant fowlpox virus encoding HIV gag/pol antigen and mutant IL-4C118 followed by booster vaccine with recombinant vaccinia virus encoding HIV gag/pol antigen and mutant IL-4C118.

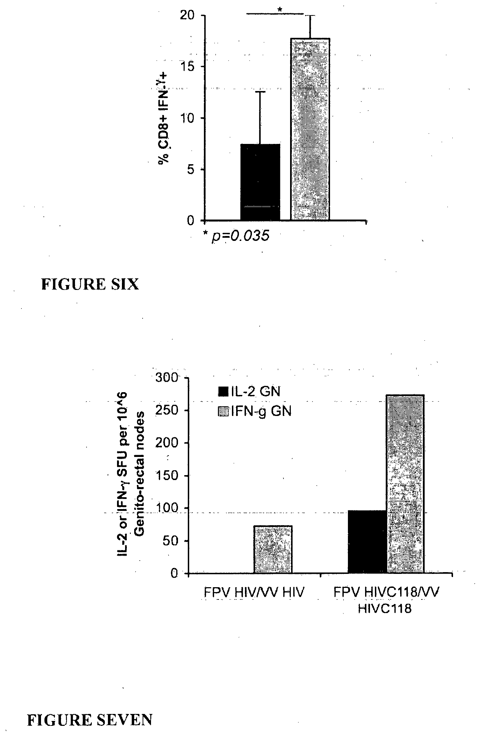

**Figure 6** is a column graph showing HIVKdGag-specific systemic (spleen) CD8⁺ T-cell immunity following co-vaccination with IL-4 receptor antagonist. Grey column = priming and booster vaccinations using recombinant viral vaccine encoding IL-4 receptor antagonist (IL-4C118) and HIV gag/pol antigens. Black column - priming and booster vaccinations using recombinant viral vaccine encoding HIV gag/pol antigens only (no IL-4 receptor antagonist).
**Figure 7** is a column graph showing HIV KdGag-specific mucosal (genito-rectal nodes) CD8⁺ T-cell immunity following IL-4 receptor antagonist (IL-4C118) prime-boost vaccination. FPV HIV IL-4C118/VV HIV IL-4C118 = priming vaccination with recombinant fowlpox virus encoding HIV gag/pol antigen and mutant IL-4C118 followed by booster vaccine with recombinant vaccinia virus encoding HIV gag/pol antigen and mutant IL-4C118; FPV HIV/VV HIV = priming vaccination with recombinant fowlpox encoding HIV gag/pol (no IL-4 receptor antagonist) followed by booster vaccination with recombinant vaccinia virus encoding HIV gag/pol (no IL-4 receptor antagonist);

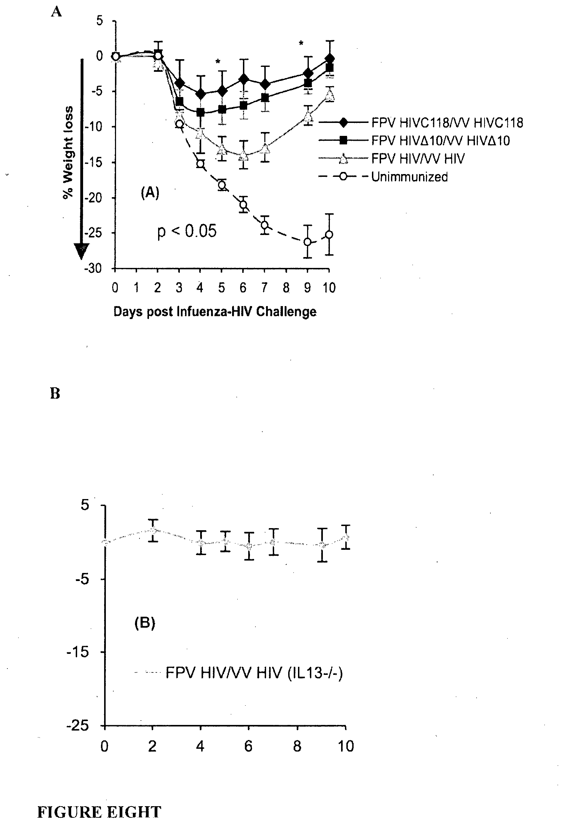

**Figure 8** provides two graphs indicating the level of protective immunity following mucosal influenza-KdGag challenge in the presence or absence of IL-4 receptor antagonist or interleukin 13 (IL-13) receptor antagonis, as assessed by weight loss and CD8⁺ T-cell responses. FPV HIV IL-4C118/VV HIV IL-4C118 = priming vaccination with recombinant fowlpox virus encoding HIV gag/pol antigen and mutant IL-4C118 followed by booster vaccine with recombinant vaccinia virus encoding HIV gag/pol antigen and mutant IL-4C 118; FPV HIV/VV HIV = priming vaccination with recombinant fowlpox encoding HIV gag/pol (no IL-4 receptor antagonist) followed by booster vaccination with recombinant vaccinia virus encoding HIV gag/pol (no IL-4 receptor antagonist); FPV HIVΔ10/VV HIVΔ10 = priming vaccination with recombinant fowlpox virus encoding HIV gag/pol antigen and IL-13Ra2 soluble receptor followed by booster vaccination with recombinant vaccinia virus encoding HIV gag/pol antigen and IL-13Ra2 soluble receptor; FPV HIV/VV HIV (IL 13 -/-) = priming vaccination with recombinant fowlpox encoding HIV gag/pol (no IL-4 receptor antagonist) followed by booster vaccination with recombinant vaccinia virus encoding HIV gag/pol (no IL-4 receptor antagonist) in IL-13 knockout mice; (n = 6-14 per group; error bar = SEM).

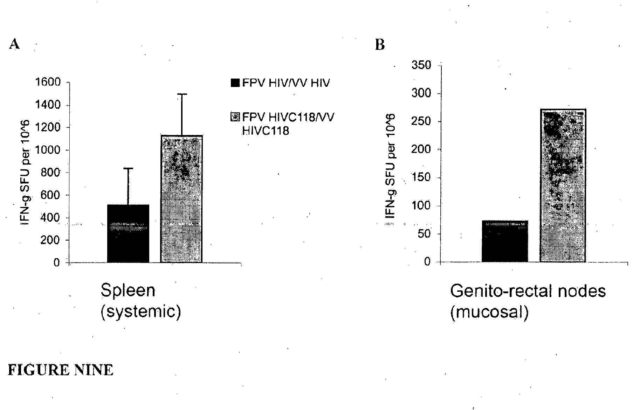

**Figure 9** provides two graphs showing memory systemic and mucosal HIV-specific T cell responses measured by ELISpot at 8 weeks post booster immunisation. FPV HIV/VV HIV = priming vaccination with recombinant fowlpox encoding HIV gag/pol (no IL-4 receptor antagonist) followed by booster vaccination with recombinant vaccinia virus encoding HIV gag/pol (no IL-4 receptor antagonist); FPV HIV IL-4C118/VV HIV IL-4C118 = priming vaccination with recombinant fowlpox virus encoding HIV gag/pol antigen and mutant IL-4C118 followed by booster vaccine with recombinant vaccinia virus encoding HIV gag/pol antigen and mutant IL-4C118.

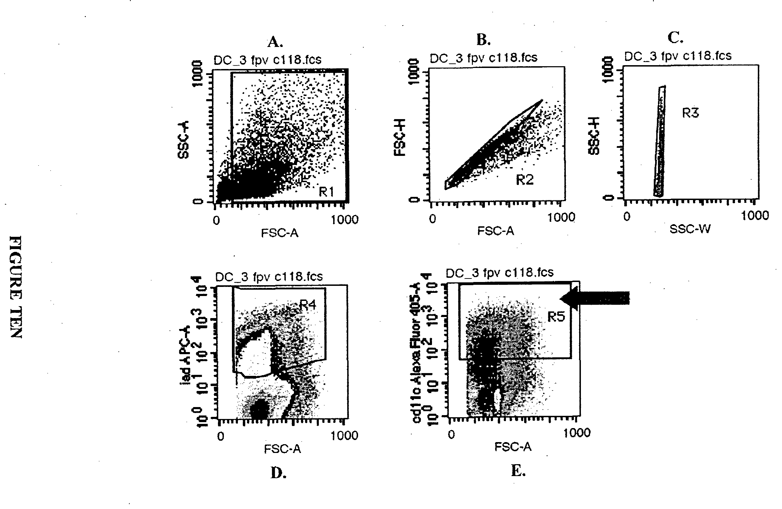

**Figure 10** provides a series of dotplots setting out an antigen presenting cell (APC) subset gating strategy used in the evaluation of APC subsets in the lung mucosae 24 hours post-priming vaccination. R1 = total APC subsets; R2 = forward scatter (FSC) doublet discrimination; R3 = side scatter (SSC) doublet discrimination; R5 = cells gated on MHC II+ CD11c+ = APC subset; APC = antigen presenting cell; DC = dendritic cell; CD11c = DC marker; 1A^{d} = MHC II cell marker.

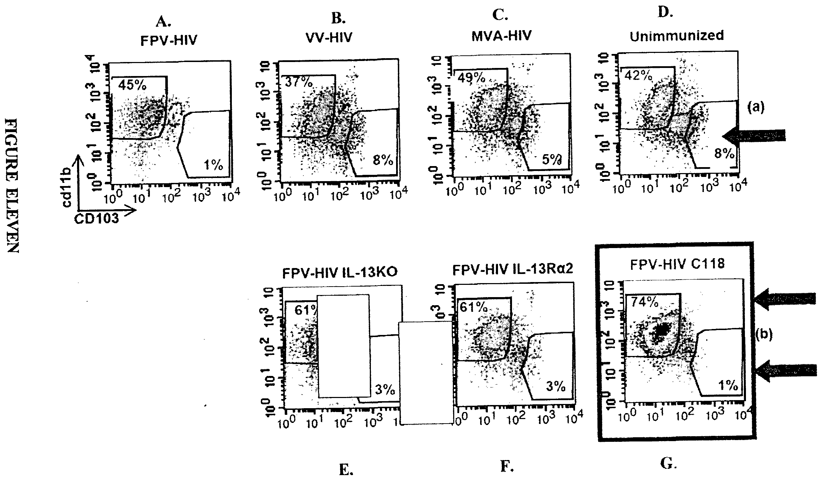

**Figure 11** provides a series of dotplots evaluating CD103 associated antigen presenting cell subsets in the lung mucosae 24h post priming vaccination. FPV-HIV = Fowl pox virus expressing HIV gag/pol genes or Fowl pox virus expressing HIV gag/pol/env genes; VV-HIV = Vaccinia virus expressing HIV gag/pol genes; MVA-HIV = Modified vaccinia ankara expressing HIV gag/pol genes; FPV-HIV IL-13KO = IL-13 knockout animals given the control vaccine FPV-HIV; FPV-HIV IL-13Rα2 = Fowl pox virus co-expressing HIV gag/pol genes and soluble IL-13Ra2 or Fowl pox virus co-expressing HIVgag/pol/ env genes and soluble IL-13Ra2; FPV-HIV C118 = Fowl pox virus co-expressing HIV gag/pol genes and IL-4 antagonist or Fowl pox virus expressing HIV gag/pol/env genes and IL-4 antagonist; CD11b = dendritic cell marker; CD103 = lung/skin-specific T cell marker; IL-13KO= IL-13-/- gene knockout mice.

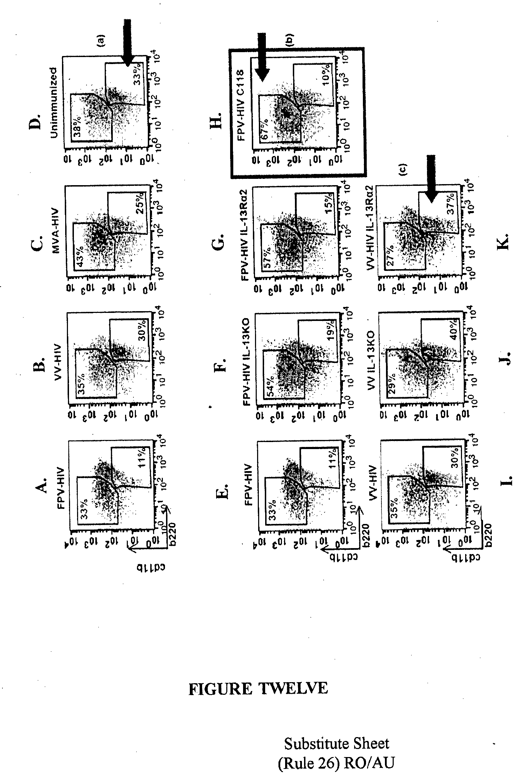

**Figure 12** provides a series of dotplots evaluating B220 associated antigen presenting cell subsets in the lung mucosae 24h post priming vaccination. FPV-HIV = Fowl pox virus expressing HIV gag/pol genes or Fowl pox virus expressing HIV gag/pol/env genes; VV-HIV = Vaccinia virus expressing HIV gag/pol genes; MVA-HIV = Modified vaccinia ankara expressing HIV gag/pol genes; FPV-HIV IL-13KO = IL-13 knockout animals given the control vaccine FPV-HIV; FPV-HIV IL-13Rα2 = Fowl pox virus co-expressing HIV gag/pol genes and soluble IL-13Ra2 or Fowl pox virus co-expressing HIVgag/pol/ env genes and soluble IL-13Ra2; FPV-HIV C118 = Fowl pox virus co-expressing HIV gag/pol genes and IL-4 antagonist or Fowl pox virus expressing HIV gag/pol/env genes and IL-4 antagonist; VV-IL13KO = IL-13 knockout animals given the control vaccine VV-HIV; VV-HIV IL-13Rα2 = Vaccinia virus co-expressing HIV gag/pol genes and soluble IL-13Ra2; CD11b = dendritic cell marker; B220 = B cell marker; C118 = IL-4 antagonist.

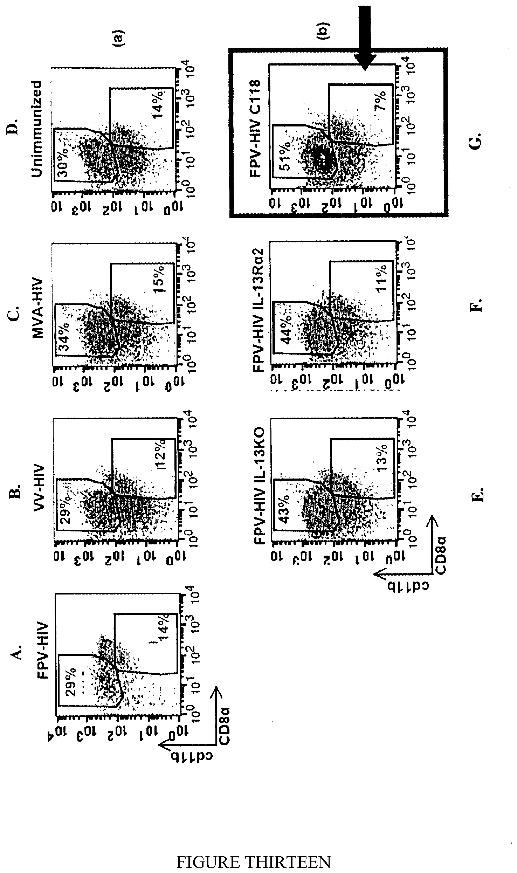

**Figure 13** provides a series of dotplots evaluating CD8-associated antigen presenting cell subsets in the lung mucosae 24h post priming vaccination. FPV-HIV = Fowl pox virus expressing HIV gag/pol genes or Fowl pox virus expressing HIV gag/pol/env genes; VV-HIV = Vaccinia virus expressing HIV gag/pol genes; MVA-HIV = Modified vaccinia ankara expressing HIV gag/pol genes; FPV-HIV IL-13KO = IL-13 knockout animals given the control vaccine FPV-HIV; FPV-HIV IL-13Rα2 = Fowl pox virus co-expressing HIV gag/pol genes and soluble IL-13Ra2 or Fowl pox virus co-expressing HIVgag/pol/ env genes and soluble IL-13Ra2; FPV-HIV C118 = Fowl pox virus co-expressing HIV gag/pol genes and IL-4 antagonist or Fowl pox virus expressing HIV gag/pol/env genes and IL-4 antagonist. CD11b = dendritic cell marker; CD8α = dendritic cell marker.

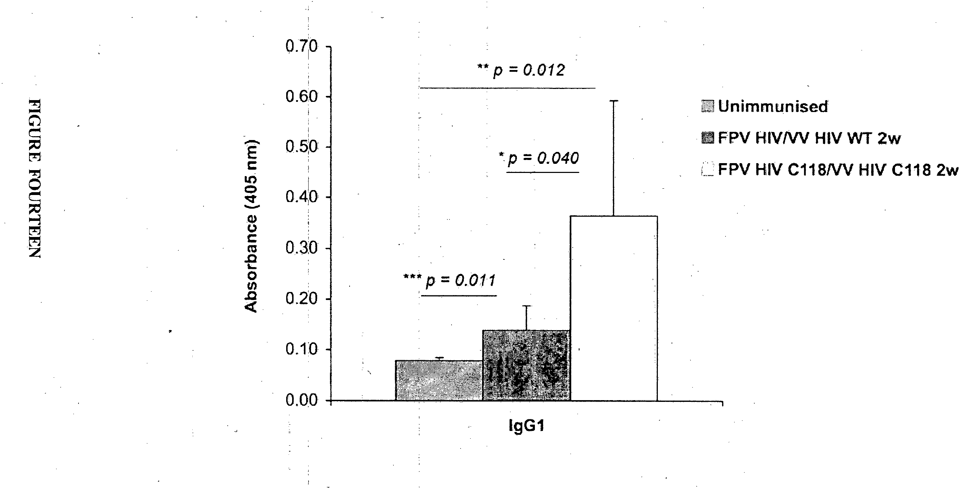

**Figure 14** is a column graph demonstrating that serum IgG1 responses to p24Gag are enhanced following HIV-IL-4R antagonist adjuvanted vaccination.

### DEFINITIONS

As used in this application, the singular form "a", "an" and "the" include plural references unless the context clearly dictates otherwise. For example, the phrase "virus" also includes a plurality of viruses.

As used herein, the term "comprising" means "including." Variations of the word "comprising", such as "comprise" and "comprises," have correspondingly varied meanings. Thus, for example, a vaccine "comprising" a recombinant virus may consist exclusively of that recombinant virus or may include one or more additional components (e.g. additional type(s) of recombinant viruses).

As used herein, the "therapeutically effective amount" includes within its meaning a non-toxic but sufficient amount of an agent or composition for use in the present invention to provide the desired therapeutic effect. The exact amount required will vary from subject to subject depending on factors such as the species being treated, the age and general condition of the subject, the severity of the condition being treated, the particular agent being administered, the mode of administration and so forth. Thus, it is not possible to specify an exact "effective amount" applicable to all embodiments. However, for any given case, an appropriate "effective amount" may be determined by one of ordinary skill in the art using only routine experimentation.

As used herein, the term "subject" includes any animal of economic, social or research importance including bovine, equine, ovine, primate, avian and rodent species. Hence, a "subject" may be a mammal such as, for example, a human or a non-human mammal.

As used herein, the term "IL-4 antagonist" encompasses any agent capable of preventing or inhibiting the production and/or biological function of IL-4 including, for example, IL-4 receptor (IL-4R) and IL-4 receptor alpha chain (IL-4Rα) antagonists.

As used herein, the terms "antagonist of IL-4R", "IL-4R antagonist", and "interleukin-4 receptor antagonist" are used interchangeably and have the same meaning. The terms encompass any agent capable of preventing or inhibiting the activation and/or continuation of a cell signaling pathway that is initiated by (i.e. commences directly *via*) IL-4Rα or a receptor complex comprising IL-4Rα. In some embodiments an IL-4R antagonist may be an agent that binds to IL-4Rα without initiating IL-4Rα-mediated cell signaling and thereby preventing or altering a binding interaction between IL-4Rα and one or more ligands (e.g. IL-4 and/or IL-13) that may otherwise be capable of activating the IL-4R receptor complex in the absence of the antagonist. In other embodiments an IL-4R antagonist may be an agent that binds to one or more ligands of IL-4Rα (e.g. IL-4 and/or IL-13) thereby blocking or altering a binding interaction between the ligand and IL-4Rα. In other embodiments, an IL-4R antagonist may be an agent capable of inhibiting or blocking a cell signaling pathway that has been initiated by (i.e. commences *via*) IL-4Rα or a receptor complex comprising IL-4Rα. For example, the agent may interact with or modify the activity of a downstream protein or molecule in a signaling pathway that is initiated by (i.e. commences *via*) IL-4Rα or a receptor complex comprising IL-4Rα.

As used herein, the term "avidity" in the context of an immune cell/immune cell population and a given antigen means the quantity or concentration of the antigen required to elicit an antigen-specific response in the immune cell/immune cell population.

As used herein, the terms "antibody" and "antibodies" include IgG (including IgG1, IgG2, IgG3, and IgG4), IgA (including IgA1 and IgA2), IgD, IgE, or IgM, and IgY, whole antibodies, including single-chain whole antibodies, and antigen-binding fragments thereof. Antigen-binding antibody fragments include, but are not limited to, Fab, Fab' and F(ab')2, Fd, single-chain Fvs (scFv), single-chain antibodies, disulfide-linked Fvs (sdFv) and fragments comprising either a VL or VH domain. The antibodies may be from any animal origin. Antigen-binding antibody fragments, including single-chain antibodies, may comprise the variable region(s) alone or in combination with the entire or partial of the following: hinge region, CH1, CH2, and CH3 domains. Also included are any combinations of variable region(s) and hinge region, CH1, CH2, and CH3 domains. Antibodies may be monoclonal, polyclonal, chimeric, multispecific, humanized, and human monoclonal and polyclonal antibodies which specifically bind the biological molecule.

As used herein, the terms "protein", "polypeptide" and "peptide" each refer to a polymer made up of amino acids linked together by peptide bonds and are used interchangeably. For the purposes of the present invention a "polypeptide" may constitute a full length protein or a portion of a full length protein.

As used herein, the term "polynucleotide" refers to a single- or double-stranded polymer of deoxyribonucleotide bases, ribonucleotide bases, known analogues of natural nucleotides, and mixtures thereof.

As used herein, the term "nucleotide sequence" will be understood to encompass DNA sequences, RNA sequences, and complementary (cDNA) sequences.

As used herein, the term "parent virus" will be understood to be a reference to a virus that can be modified to incorporate exogenous genetic material to form a recombinant virus of the present invention.

As used herein, the term "recombinant virus" will be understood to be a reference to a "parent virus" comprising at least one exogenous nucleotide sequence.

As used herein, the term "exogenous nucleotide sequence" when used in the context of a recombinant virus will be understood to encompass any nucleotide sequence inserted into the genome of a parent virus to form a recombinant virus.

As used herein, the term "immunogenic" when used in the context of a given component such as, for example, a nucleotide sequence, polypeptide, an exogenous nucleotide sequence, an exogenous polypeptide, an antigen, or an epitope, means that the agent has a capability to induce an immune response, enhance an existing immune response, or alter an existing immune response, either alone, or acting in combination with other agent(s).

It will be understood that "inducing" an immune response as contemplated herein includes inciting an immune response and enhancing a previously existing immune response.

As used herein, the term "kit" refers to any delivery system for delivering materials. Such delivery systems include systems that allow for the storage, transport, or delivery of reaction reagents (for example labels, reference samples, supporting material, etc. in the appropriate containers) and/or supporting materials (for example, buffers, written instructions for performing an assay etc.) from one location to another. For example, kits may include one or more enclosures, such as boxes, containing the relevant reaction reagents and/or supporting materials. The term "kit" includes both fragmented and combined kits.

As used herein, the term "fragmented kit" refers to a delivery system comprising two or more separate containers that each contains a subportion of the total kit components. The containers may be delivered to the intended recipient together or separately. Any delivery system comprising two or more separate containers that each contains a subportion of the total kit components are included within the meaning of the term "fragmented kit".

As used herein, a "combined kit" refers to a delivery system containing all of the components of a reaction assay in a single container (e.g. in a single box housing each of the desired components).

It will be understood that use the term "about" herein in reference to a recited numerical value includes the recited numerical value and numerical values within plus or minus ten percent of the recited value.

It will be understood that use of the term "between" herein when referring to a range of numerical values encompasses the numerical values at each endpoint of the range. For example, a polypeptide of between 10 residues and 20 residues in length is inclusive of a polypeptide of 10 residues in length and a polypeptide of 20 residues in length.

Any description of prior art documents herein, or statements herein derived from or based on those documents, is not an admission that the documents or derived statements are part of the common general knowledge of the relevant art.

For the purposes of description all documents referred to herein are hereby incorporated by reference in their entirety unless otherwise stated.

### DETAILED DESCRIPTION

The following detailed description conveys exemplary embodiments of the present invention in sufficient detail to enable those of ordinary skill in the art to practice the present invention. Features or limitations of the various embodiments described do not necessarily limit other embodiments of the present invention or the present invention as a whole. Hence, the following detailed description does not limit the scope of the present invention, which is defined only by the claims.

Vaccines capable of eliciting high-avidity T-lymphocyte responses and/or a sizeable magnitude of T lymphocyte responses against viral antigens are highly sought after in the field. Given that mucosal surfaces are the first portals of entry for viruses, vaccines capable of eliciting responses at these sites are also desirable. The present inventors have identified that the avidity and/or magnitude of antigen-specific T-lymphocyte responses can be significantly enhanced by inhibiting IL-4, that is, by reducing the availability of IL-4 and/or inhibiting IL-4 function. This may be done transiently, such that normal IL-4 levels and function can be restored in the long term.

Accordingly, some aspects of the present invention relate to compositions, vaccines and medicaments capable of enhancing the magnitude of immune cell responses and/or enhancing immune cell avidity for a targeted infectious agent. The compositions may comprise an immunogenic component capable of inducing immunity against the infectious agent together with a component capable of inhibiting the production and/or activity of IL-4. Alternatively, the immunogenic component and component capable of inhibiting the production and/or activity of IL-4 may be provided in separate compositions for simultaneous or sequential administration. Compositions of the present invention may be provided in kits.

Other aspects of the present relation relate to methods for inducing antigen-specific immune responses in a subject. The methods comprise administering one or more compositions of the invention, and may be effective in increasing the avidity of immune cells for the antigen in a subject and/or increasing the number of immune cells specific for the antigen.

Still other aspects of the present invention relate to methods for preventing and/or treating infection by administration of the compositions, vaccines and medicaments provided herein. The methods may involve administration of preventative and/or therapeutic vaccines.

### Interleukin-4 (IL-4) antagonists

As known to those skilled in the field, IL-4 is an immunoregulatory cytokine capable of binding two separate receptor complexes. The type I receptor complex comprises the interleukin-4 receptor alpha (IL-4Rα) chain and the common gamma chain (yc). The type II receptor complex comprises the IL-4Rα chain but the yc is substituted by the IL-13 protein receptor alpha 1 (IL-13Rα1) chain or the IL-13 protein receptor 2 (IL-13Rα2) chain. In either case, only the IL-4Rα chain is capable of binding IL-4, and heterodimerisation of the IL-4Rα chain with yc, IL-13Rα1, or IL-13Rα2 is also required to initiate cell signalling (via the JAK/STAT pathway in the case of IL-4Rα/γc and IL-4Rα/IL-13Rα1.

The compositions and methods of the present invention employ antagonists of IL-4 production and/or function. As demonstrated in the Examples of the present specification, the IL-4 antagonists have been found to significantly increase/enhance the magnitude and/or avidity of immune cell responses for a co-administered antigen. Without being limited by theory, it is postulated that inhibition of IL-4 production and/or function in the local milieu of an immune response reduces inhibitory effects on antigen-specific CTL production and/or function. Accordingly, when co-administered with a particular antigen the IL-4 antagonists provide a greater magnitude and/or avidity of antigen-specific cells (e.g. antigen specific CD8⁺ T lymphocytes).

An IL-4 antagonist as contemplated herein encompasses any agent capable of preventing or inhibiting the production and/or biological function of IL-4. Given that many of the biological functions of IL-4 are mediated through binding interactions with IL-4R, many antagonists of IL-4 production and/or function are also antagonists of IL-4R (and *vice versa*).

The IL-4 antagonist may inhibit the production and/or biological function of IL-4 by virtue of a binding interaction with IL-4. The binding interaction between the antagonist and IL-4 may prevent IL-4 from binding to or otherwise interacting with other molecule(s) (e.g. IL-4Rα) and thereby inhibit IL-4 function and/or production. In this case, the affinity of the binding interaction between IL-4 and the antagonist may be higher than that of the binding interaction between IL-4 and the other molecule(s) to which it may otherwise bind. By way of non-limiting example only, soluble forms of the IL-4 receptor may be used as IL-4 antagonists. These may be mutated so as to bind to IL-4 with high affinity (e.g. a soluble IL-4R exclusively composed of the extracellular domain of the IL-4Rα chain). Antibodies may also be used as IL-4 antagonists. The antibodies may have binding specificity the IL-4R (e.g. IL-4Rα), or, a ligand of IL-4Rα (e.g. IL-4).

Additionally or alternatively, the IL-4 antagonist may inhibit the production and/or biological function of IL-4 by preventing or inhibiting the expression and/or function of any one or more downstream proteins or molecules in a signaling pathway that is initiated by (i.e. commences *via*) IL-4R. For example, the agent may interact with or modify the activity of a downstream protein or molecule in a signaling pathway that is initiated by (i.e. commences *via*) IL-4R or a receptor complex comprising IL-4Rα.

Additionally or alternatively, the IL-4 antagonist may inhibit the production and/or biological function of IL-4 by virtue of a binding interaction with the IL-4R or a component thereof (e.g. IL-4Rα). In this case, the binding interaction will generally not initiate cell signalling mediated through IL-4R, but the binding affinity will generally be sufficient to prevent other ligands binding to the IL-4R or component thereof (e.g. IL-4Rα), or the capacity to bind in a manner that is capable of initiating cell signalling via IL-4R.

By way of non-limiting example, the IL-4 antagonist may be one which binds to IL-4R or a component thereof (e.g. IL-4Rα). These IL-4 antagonists are therefore also IL-4R antagonists and IL-4Rα antagonists. The binding interaction generally does not induce significant levels of cell signalling, or, induce any cell signalling, via the IL-4R, but the affinity of the interaction is of sufficient strength to prevent other ligands of the IL-4R (e.g. IL-4 and/or IL-13) from either binding to the IL-4R (e.g. to IL-4Rα), or the capacity to bind in a manner that is capable of initiating cell signalling via IL-4R.

In certain embodiments, the IL-4 antagonist binds to IL-4Rα (and is thus also an antagonist of IL-4Rα). Any IL-4 antagonist that binds to IL-4Rα without substantially instigating cell signalling, whilst preventing interactions between IL-4Rα (or an IL-4R comprising IL-4Rα) and a ligand thereof may be used in compositions and methods of the present invention. Antagonists of this category are well known to persons skilled in the field and include, for example, modified ligands of IL-4Rα (e.g. mutant IL-4 molecules) that are capable of binding to IL-4Rα without initiating cell signalling, thus blocking IL-4R function. Specific and non-limiting examples of modified IL-4Rα ligands include IL-4 with C-terminal mutations such as the deletion of one or more C terminus residues (e.g. deletion or substitution of tyrosine residue 119 in the secreted form of murine IL-4 such as IL-4C118 as described herein) and equivalent mutations in other mammalian IL-4 homologues; deletion or substitution of tyrosine at position 124 in the secreted form of human or macaque IL-4 (e.g. IL-4C123 as described herein) and equivalent mutations in other mammalian IL-4 homologues; splice variants such as those with a deletion of residue(s) encoded by exon 2 of the IL-4 gene (e.g. IL-4 delta 2 in humans and equivalent mutations in other mammalian IL-4 homologues); and mutations at positions 121 and/or 124 in the mature secreted form of the human IL-4 protein and equivalent positions in other mammalian IL-4 homologues (e.g. the IL-4 Y124D mutant or the R121D/Y124D IL-4 mutant in humans; the Q116D/Y119D IL-4 mutant or the Y119D mutant in mice). It is also contemplated that IL-4Rα-specific antibodies could also be designed to bind to IL-4Rα without substantially instigating cell signalling.

Numerous methods are available to determine IL-4R binding interactions and/or IL-4R-mediated cell signaling, and are well known to those in the field. Non-limiting examples include surface plasmon resonance, fluorescence resonance energy transfer (TR-FRET), chemical co-immunoprecipitation, bioluminescence resonance energy transfer (BRET), IL-4-dependent STAT6 activation measured by electrophoretic mobility shift assay (EMSA), and the like.

### Antigens

The compositions and methods of the present invention employ antigens for stimulating immune responses. The antigen-specific immune response induced is enhanced by the presence of an IL-4 antagonist (e.g. an IL-4Rα antagonist).

The antigen may be any molecule capable of stimulating an immune response in a subject (e.g. proteins, peptides, polysaccharides and the like), including endogenous (self) antigens and exogenous (foreign) antigens. Combinations of antigens may be utilised.

In certain embodiments, the antigen is a peptide antigen. The peptide antigen may be of a size that is suitable for stimulating an immune response to the target antigen of interest. The size of the peptide used may be optimised for T cell and/or B cell epitope processing requirements. Those of skill in the field will recognise that major histocompatibility complex class I-restricted T cell epitopes are generally between 8 and 10 amino acid residues long, whereas class II-restricted T cell epitopes are generally between 12 and 25 amino acid residues long. Flanking residues may be included in either case for optimal proteolytic processing (e.g. 2-3 natural flanking amino acid residues for a class I-restricted T cell epitope). Class II-restricted epitopes may contain a central 9-10 amino acid residue core that binds specifically to class II MHC molecules, with flanking residues on either side of the core serving to stabilise binding.

A linear B cell epitope may be at least: 6, 7, 8, 9, 10, 11, 12, 13, 14, 15, 20, 25 or 30 amino acid residues. The size of the B cell epitope may be less than about: 500, 200, 100, 80, 60, 50, or 40 amino acid residues in length.

The size of the peptide used in a composition or method of the present invention may be suitable for presentation of an epitope contained within the peptide by an antigen-presenting cell to a T lymphocyte and/or a B lymphocyte.

Techniques for identifying and selecting effective antigenic peptides (e.g. minimal peptide sequences capable of eliciting an immune response) are well known to those skilled in the field. By way of non-limiting example only, reference is made to Rappuoli and Bagnoli (Eds), (2011), "Vaccine Design: Innovative Approaches and Novel Strategies", Caister Academic Press, UK; and Agudelo and Patarroyo, (2010), "Quantum chemical analysis of MHC-peptide interactions for vaccine design", Mini Rev Med Chem; 10(8):746-58, the entire contents of which are incorporated herein by reference.

In some embodiments, the antigens may be viral antigens. Exemplary viral components from which the antigens may be derived include envelopes, coats, capsules, capsids, toxins, RNA, DNA and combinations thereof. No particular limitation exists in relation to the particular type of virus from which the antigen may be derived. Non-limiting examples include herpesviruses (e.g. human herpesvirus (HHV) types 1-8) such as herpes simplex virus types I and II, cytomegalovirus, varicella zoster virus, epstein barr virus; hepatitis viruses (e.g. hepatitis A, B, C); orthomyxoviruses (e.g. influenza virus types A, B, C and recombinant forms thereof); flaviviruses (e.g. dengue virus, west nile virus, Japanese encephalitis virus, yellow fever virus); coronaviruses (e.g. SARS virus); paramyxoviruses (e.g. hendra virus, measles virus, sendai virus, mumps virus, respiratory syncitial virus); enteroviruses (e.g. coxsackieviruses, polioviruses, rhinoviruses); togaviruses (e.g. rubellavirus); adenoviruses (e.g. types 4 and 7); papillomaviruses (e.g. human papillomavirus); filoviruses (e.g. ebola virus); bunyaviruses (e.g. hantavirus, phlebovirus); rhabdoviruses (e.g. vesicular stomatisis virus; lyssaviruses such as the rabies virus); and retroviruses (e.g. lentiviruses such as HIV-1, HIV-2, SIV and visna virus).

Any suitable antigen from a given virus may be used. Non-limiting examples of herpes simplex viral antigens include immediate early proteins, glycoprotein B, glycoprotein D and VZV antigens 9PI and gpII. Non-limiting examples of antigens from hepatitis viruses include the S, M, and L envelope proteins and pre-S surface antigen. Non-limiting examples of suitable influenza virus antigens include haemagglutinin, neuraminidase and other influenza virus envelope proteins. Non-limiting examples of Japanese encephalitis viral antigens include proteins E, M-E, M-E-NS 1, NS 1, and NS 1-NS2A. Non-limiting examples of measle virus antigens include the measle virus fusion protein. Exemplary antigens of the rubella virus include, but are not limited to, proteins E1 and E2. Exemplary rotaviral antigens included VP7sc. Non-limiting examples of respiratory syncytial viral antigens include the RSV fusion protein and the M2 protein. Exemplary rabies virus antigens include, but are not limited to, the rabies virus glycoprotein and the rabies virus nucleoprotein. Non-limiting examples of papillomavirus antigens include the L1 and L2 capsid proteins and the E6/E7 antigens associated with cervical cancers.

In certain embodiments, the viral antigen is from a retrovirus. The retrovirus may be a human immunodeficiency virus (e.g. HIV-1 or HIV-2). Non-limiting examples of suitable antigens from HIV include those in gene products of the gp120 env, gp140 env, gp160 env, p18, gag, pol, vif, vpr, vpu, tat, rev, and nef genes, and combinations thereof. For example, the antigen may be an HIV-1 gag, pol, env, gag/pol, or gag/pol/env gene product.

### Compositions

Compositions of the present invention may comprise an immunogenic component (e.g. an antigen) capable of inducing immunity against a targeted agent together with a component capable of inhibiting the production and/or activity of IL-4.

Alternatively, the immunogenic component (e.g. antigen) and component capable of inhibiting the production and/or activity of IL-4 may be provided in separate compositions formulated for simultaneous or sequential administration.

### - polypeptides and proteins

Compositions of the present invention may comprise an IL-4 antagonist (e.g. an IL-4Rα antagonist) and/or an immunogenic component (e.g. an antigen) capable of inducing immunity against a targeted agent.

The compositions may be formulated for injection, inhalation or topical administration facilitating direct exposure of host cells and tissues to the antagonist and/or immunogenic component. In certain embodiments, the antagonist and/or immunogenic component may be provided in nanoparticles (see, for example, methodologies described in US patent nos. 7,611,690, 7,858,596, and 8,048,404). Additionally or alternatively, the compositions may be formulated in dry powder form suitable for delivery by particle bombardment (see, for example, methods relying on gas-driven particle acceleration such as those described in US patent nos. 5,584,807 5,865,796, and 6,010,478; and methods relying on gas-driven needleless injection such as those described in US patent nos. 5,299,163, 5,383,851 and 5,993,412).

### - nucleic acid constructs

Compositions of the present invention may comprise nucleic acid constructs capable of expressing polynucleotide(s) encoding an IL-4 antagonist and/or an immunogenic component (e.g. an antigen) capable of inducing immunity against a targeted agent. The polynucleotide encoding the IL-4 antagonist may be in the same or a different construct to the polynucleotide encoding the immunogenic component. The nucleic acid construct may be, for example, an expression vector, a plasmid vector, a viral vector, a phosmid, a cosmid, a recombinant virus or any other vector construct suitable for the insertion of foreign sequences, introduction into cells and subsequent expression of the introduced sequences.

The construct may be a replicon particle-based vaccine vector. Non-limiting examples include alphavirus replicon particle-based vaccine vectors derived from Sindbis virus (SIN), Semliki Forest virus (SFV), and Venezuelan equine encephalitis virus (VEE).

The polynucleotides encoding the IL-4 antagonist and/or immunogenic component (e.g. an antigen) may be operably linked to regulatory polynucleotide sequences (e.g. transcriptional and/or translational control sequences such as a promoter sequence, 5' untranslated region, 3' untranslated region, c/s-regulatory region, ribosomal-binding sequences, transcription and translation start sites, and the like). The promoters may be constitutive or inducible. The regulatory polynucleotide sequences may be compatible with for expression in the cell or tissue type for which the construct is intended for administration.

A promoter will be understood to mean a DNA sequence recognised by a cell's transcriptional machinery to initiate transcription of a downstream polynucleotide sequence. Non-limiting examples of suitable promoters include: constitutive promoters such as those found in some eukaryotic viruses such as the adenovirus, polyoma virus, CMV (e.g. cytomegalovirus immediate early gene promoter), SV40, Rous sarcoma virus (e.g. long terminal repeat promoter), avian sarcoma virus, herpes simplex virus thymidine kinase promoter, hepatitis B virus retroviral LTR regions; promoters present in eukaryotic genes such as: the EF-1 alpha promoter, metalothioneine gene promoter, actin promoter, and the immunoglobulin promoter; inducible promoters requiring the addition of a substance or an exogenous signal for expression such as the tetracycline promoter, NFKappaB/UV light, Cre/lox, heat shock promoters, regulatable RNA polymerase II promoters (see PCT publication No. WO/2006/135436); tissue-specific promoters such as the PSA promoter described in PCT publication No. WO/2006/012221; and constitutive RNA polymerase III promoters such as RNA pol III promoters from the 5S ARN, ARN 7SL ARNt, ARNsn, H1 and U6 genes. Other non-limiting examples include the human elongation factor 1α promoter, and the human ubiquitin c promoter.

The nucleic acid constructs may be formulated for delivery to target cells and tissues using any suitable method known in the field.

In certain embodiments, the constructs may be formulated for delivery in the form of naked DNA (see, for example, techniques described in US patent nos. 6,265,387, 6,972,013, and 7,922,709).

### - recombinant viruses

In some embodiments, a nucleic acid construct included in a composition of the present invention may be in the form of a recombinant virus. The virus may be genetically modified to express a polynucleotide encoding one or more exogenous proteins or exogenous protein components. For example, the recombinant virus may be engineered so as to express a polynucleotide encoding an IL-4 antagonist (e.g. an IL-4Rα antagonist) and/or an immunogenic component comprising one or more antigens.

Recombinant viruses of the present invention may be produced by modification of a "parent" virus to incorporate exogenous genetic material. Reference herein to a specific type of recombinant virus (e.g. a recombinant fowlpox, vaccinia or poxvirus) denotes a parent virus of the indicated type that has been modified to incorporate exogenous genetic material.

No particular limitation exists regarding the specific type of parent virus used to generate recombinant viruses of the present invention. Non-limiting examples of suitable parent viruses include retroviruses (e.g. lentiviruses such as HIV, HIV-1, HIV-2, FIV, BIV, EIAV, MW, CAEV, and SIV), adenoviruses and adeno-associated viruses, alphaviruses (e.g. VEE), flaviviruses, and poxviruses (e.g. vaccinia viruses, avian pox viruses (such as fowlpox virus), and avipox viruses).

The recombinant viruses may be live or live-attenuated recombinant viruses. In general, the recombinant viruses are replication-competent meaning that they are capable of reproducing in a host cell which they have infected.

Recombinant viruses of the present invention comprise at least one exogenous nucleotide sequence. It will be understood that in the context of the present invention, a nucleotide sequence encompasses DNA, RNA, and complementary (cDNA) sequences.

An exogenous nucleotide sequence as used herein encompasses any nucleotide sequence inserted into the genome of a parent virus to form a recombinant virus. In certain embodiments, the exogenous nucleotide sequence encodes an IL-4 antagonist (e.g. an IL-4Rα antagonist) and/or an immunogenic molecule (e.g. an antigen) from a different microorganism (e.g. a different virus).

Without imposing any specific limitation on the length the exogenous nucleotide sequence, in some embodiments the sequence may be between about 10 to 15, 15 to 25, 20 to 30, 25 to 35, 30 to 40, 35 to 45, 40 to 50, 50 to 75, 75 to 100, 100 to 150, 150 to 200, 200 to 275, 275 to 350, 350 to 500, 500 to 750, 750 to 1000, 1000 to 1250, 1250 to 1500, 1500 to 1750, 1750 to 2000, 2000 to 2500, 2500 to 3000, or more than 3000 nucleotides in length.

A recombinant virus of the present invention may comprise multiple exogenous polynucleotide sequences, including duplicate(s) of the same exogenous sequence and/or combinations of different exogenous sequences (e.g. a polynucleotide encoding an IL-4 antagonist (e.g. an IL-4Rα antagonist) and a polynucleotide encoding an immunogenic component comprising one or more antigens.

No particular limitation exists regarding the location at which the exogenous polynucleotide sequence(s) is/are inserted into the virus genome, which will depend primarily on the particular polynucleotide(s) and the parent virus. However, it is preferred that the exogenous sequence is inserted at a location in the viral genome that minimises adverse effects on virus function, replication, and/or proteolytic processing of translated viral polypeptides.

An exogenous polynucleotide may be inserted into an open reading frame (ORF) of the virus. For example, the polynucleotide may be inserted at a junction between two viral genes. Alternatively, the polynucleotide may be inserted into the viral genome in a region that is not in an ORF. For example, the polynucleotide may be inserted into non-coding sequence between two different ORFs, 5' to an ORF (e.g. in the 5'-untranslated region (5'-UTR)), or 3' to an ORF (e.g. in the 3'-untranslated region (3'-UTR)).

An exogenous polynucleotide inserted into a parent virus may comprise at least one nucleotide sequence encoding a proteolytic cleavage site. The proteolytic cleavage site may be advantageous in facilitating cleavage and release of the encoded polypeptide from other viral-encoded polypeptides if translated as components of a single polypeptide. Suitable sequences encoding proteolytic cleavage sites and methods for their incorporation into other sequences are well known in the art and described in standard texts.

Recombinant viruses of the present invention may comprise at least one endogenous or exogenous nucleotide sequence for initiating translation of the exogenous sequence (e.g. a 5' cap, internal ribosome entry site, and the like) from the parent virus and/or from a different foreign virus.

An exogenous polynucleotide inserted into a parent virus may comprise a signal peptide for directing transport of an encoded IL-4 antagonist and/or immunogenic component (e.g. an antigen) within an infected cell (e.g. to the endoplasmic reticulum) and/or out of the infected cell.

Recombinant viruses according to the present invention may be generated using standard molecular biology techniques and recombinant nucleic acid technologies known to those skilled in the field. The particular method utilised will depend on the parent virus used and the particular exogenous polynucleotide(s) to be inserted. The insertion of exogenous nucleotide sequences into the parent virus can be accomplished using standard techniques of molecular biology. Suitable techniques are described, for example, in standard texts including Sambrook et al., (1989), "Molecular Cloning: A Laboratory Manual", (2nd ed., Cold Spring Harbor Laboratory Press, Plainview, New York; and Ausubel et al. (Eds), (2000-2010), "Current Protocols in Molecular Biology", John Wiley and Sons, Inc.. The Examples of the present specification also provide specific guidance on methods for the insertion of exogenous nucleotide sequences into a parent virus. Using these principles, any of a variety of exogenous nucleotide sequences may be inserted into a parent virus genome.

By way of non-limiting example only, genetic recombination may be used to transfer exogenous polynucleotides from a vector to a parent virus by homologous or heterologous recombination events. The exogenous polynucleotide may be provided in a vector flanked by sequence from the parent virus. The exogenous polynucleotide or flanking sequence may comprise a selectable marker. Upon infection of a host cell by the vector and parent virus, recombination events can serve to replace endogenous sequence of the parent virus with exogenous sequences from the vector. Recombinant viruses so generated may be selected using the selectable marker.

Additionally or alternatively, a recombinant viral complementary DNA (cDNA) construct may be generated comprising a cDNA copy of the parent virus genome with one or more exogenous cDNA sequences inserted at target position(s) in the genome. This may be achieved, for example, by generating cDNA fragments of the viral genome via polymerase chain reaction (PCR) and/or cloning plasmids comprising the fragments. Various genomic fragments may be joined together using, for example, appropriate restriction sites to cleave genomic cDNA fragments to produce, for example, overhanging ends that can be ligated to ends of other fragments. In this way, a cDNA copy of the viral genome can be constructed with exogenous cDNA sequences inserted at desired position(s) along with an appropriately positioned promoter sequence for an RNA polymerase. An RNA copy of the recombinant cDNA construct may then be generated using an appropriate RNA polymerase. The viral RNA transcript may then be transfected into an appropriate cell line (e.g. BHK-21 cells or Vero cells) using an appropriate technique (e.g. electroporation), wherein production of recombinant viruses may then occur.

Additional non-limiting examples of suitable methodologies to prepare recombinant viruses from parent strains include retroviral systems such as those disclosed in US patent nos. 5,219,740, 5,219,740, 7,250,299 and 7,608,273, alphavirus systems such as those described in US patent nos. 6,465,634 and 7,811,812, flavivirus systems such as those described in US patent nos. 5,744,140 and 8,124,398, adeno-associated virus systems such as those described in US patent nos. 5,173,414, 7,022,519, and 7,125,705, adenovirus-based systems such as those described in US patent nos. 6,905,862, 7,989,425, and 6,468,771, poxvirus systems such as those described in US patent nos. 7,015,024 and 7,338,662, and avipox virus systems such as those described in US patent nos. 5,871, 742 and 6,340,462.

### - exemplary recombinant viruses

Certain embodiments of the present invention relate to compositions comprising recombinant poxviruses (i.e. poxviridae family), and in particular those of the Chordopoxvirinae subfamily of poxviruses. The use of such viruses may be advantageous in that their genomes can accommodate incorporation of large foreign DNA sequences. This in turn makes it possible to engineer these viruses to express heterologous gene sequences encoding full proteins (e.g. IL-4 antagonists including IL-4Rα receptor antagonists and/or immunogenic peptides comprising antigens). The use of these viruses may also be advantageous due to favourable safety profiles in animals including humans.

By way of non-limiting example only, attenuated vaccinia viruses, such as modified vaccinia virus Ankara (MVA), and NYVAC, may be used as parent strains to create recombinant poxviruses. The parent viruses may have had one or more pathogenic genes removed or inactivated during their generation.

Additionally or alternatively, host-restricted strains such as avipoxvirus vectors based on canarypox virus vector (ALVAC) or fowlpox (TROVAC) may be used as parent strains to create recombinant poxviruses.

The recombinant poxviruses may be utilised in compositions of the present invention so as to provide a capability to express an IL-4 antagonist (e.g. an IL-4Rα receptor antagonist) in a transient manner, for example, at a localised site of immunisation. When co-administered with an immunogenic component such as an antigen (e.g. encoded in the same or a separate poxvirus), the IL-4 antagonist may enhance the induction of antigen-specific immune cells (e.g. CD8⁺ T lymphocytes) with high avidity. Immune responses developed against the recombinant poxvirus will eventually clear the viruses, thus meaning that the IL-4 antagonist is expressed transiently rather than permanently, improving the safety profile.

In exemplary embodiments, compositions of the present invention may comprise one or a series of recombinant vaccinia and/or fowlpox viruses. The recombinant virus(es) may comprise exogenous polynucleotide sequence(s) encoding an IL-4 antagonist (e.g. an IL-4Rα receptor antagonist), at least one antigen from an infectious microorganism (e.g. a pathogenic virus), or both. The antigen may be a viral antigen (e.g. a retroviral antigen). The antigen may be an antigen from HIV-1 and/or HIV-2. The antigen may be encoded by any one or more of the gp120 env, gp140 env, gp160 env, p18, gag, pol, vif, vpr, vpu, tat, rev, and nef genes, or combinations thereof. The IL-4 antagonist may be a mutant form of IL-4 comprising a mutation that renders it capable of binding to IL-4Rα without initiating cell signalling. For example, the mutation(s) may be at residue 121 and/or 124 (e.g. R121D and/or Y124D of the mature secreted human form of IL-4) or a corresponding position in an IL-4 homolog from another species. The mutation(s) may be a deletion of some or all residues encoded by exon 2 of the IL-4 gene.

### - formulations, medicaments and vaccines

Compositions and medicaments of the present invention may be prepared using methods known to those of ordinary skill in the art. Non-limiting examples of suitable methods are described in Gennaro et al. (Eds), (1990), "Remington's Pharmaceutical Sciences", Mack Publishing Co., Easton, Pennsylvania, USA.

Compositions and medicaments of the present invention may comprise a pharmaceutically acceptable carrier, excipient, diluent and/or adjuvant. "Pharmaceutically acceptable" carriers, excipients, diluents and/or adjuvants as contemplated herein are substances which in general do not produce significant adverse reaction(s) when administered to a particular recipient such as a human or non-human animal. Pharmaceutically acceptable carriers, excipients, diluents, and adjuvants are generally also compatible with other ingredients of the composition or medicament. Non-limiting examples of suitable excipients, diluents, and carriers can be found in the *"*Handbook of Pharmaceutical Excipients" 4th Edition, (2003) Rowe et al. (Eds), The Pharmaceutical Press, London, American Pharmaceutical Association, Washington.

Compositions and medicaments of the present invention may be formulated as vaccines. Vaccines of the present invention include both preventative vaccines (i.e. vaccines administered for the purpose of preventing infection) and therapeutic vaccines (i.e. vaccines administered for the purpose of treating infection). A vaccine of the present invention may therefore be administered to a recipient for prophylactic, ameliorative, palliative, or therapeutic purposes.

Compositions and medicaments of the present invention may be in a form suitable for administration by injection, in a formulation suitable for oral ingestion (such as, for example, capsules, tablets, caplets, elixirs), in the form of an ointment, cream or lotion suitable for topical administration, in a form suitable for delivery as an eye drop, in an aerosol form suitable for administration by inhalation, such as by intranasal inhalation or oral inhalation, or in a form suitable for parenteral administration, that is, subcutaneous, intramuscular or intravenous injection.

Supplementary active ingredients such as adjuvants or biological response modifiers can also be incorporated into compositions and medicaments of the present invention. Although adjuvant(s) may be included in pharmaceutical compositions of the present invention they need not necessarily comprise an adjuvant. In such cases, reactogenicity problems arising from the use of adjuvants may be avoided.

In general, adjuvant activity in the context of a composition or medicament of the present invention includes, but is not limited to, an ability to enhance the immune response (quantitatively or qualitatively) induced by immunogenic components in the composition or medicament (e.g. an antigen). This may reduce the dose or level of the immunogenic components required to produce an immune response and/or reduce the number or the frequency of immunisations required to produce the desired immune response.

Any suitable adjuvant may be included in a composition or medicament of the present invention. For example, an aluminium-based adjuvant may be utilised. Suitable aluminium-based adjuvants include, but are not limited to, aluminium hydroxide, aluminium phosphate and combinations thereof. Other specific examples of aluminium-based adjuvants that may be utilised are described in European Patent No. 1216053 and United States Patent No. 6,372,223. Other suitable adjuvants include Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminium salts such as aluminium hydroxide gel (alum) or aluminium phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A; oil in water emulsions including those described in European Patent No. 0399843, United States Patent No. 7,029,678 and PCT Publication No. WO 2007/006939; and/or additional cytokines, such as GM-CSF or interleukin-2, -7, or -12, granulocyte-macrophage colony-stimulating factor (GM-CSF), monophosphoryl lipid A (MPL), cholera toxin (CT) or its constituent subunit, heat labile enterotoxin (LT) or its constituent subunit, toll-like receptor ligand adjuvants such as lipopolysaccharide (LPS) and derivatives thereof (e.g. monophosphoryl lipid A and 3-Deacylated monophosphoryl lipid A), muramyl dipeptide (MDP) and F protein of Respiratory Syncytial Virus (RSV).

### Methods

The present invention provides methods for inducing antigen-specific immune responses in a subject. The methods may be effective for increasing the avidity of immune cells for an administered antigen and/or increasing the number of immune cells specific for an administered antigen in a subject. The methods may be conducted for prophylactic, ameliorative, palliative, and/or therapeutic purposes. For example, the methods may be used for preventing an infection in a subject (i.e. vaccination) and/or treating an infection in a subject (e.g. viral infections, retroviral infections, HIV-1 and/or HIV-2 infection).

The methods require administration of one or more compositions, medicaments or vaccines of the present invention. Accordingly, the methods require the administration of an IL-4 antagonist such as, for example, any one or more of those described in the section above entitled "Interleukin-4 (IL-4) antagonists". For example, the methods may comprise administering an antagonist that binds to IL-4, or an IL-4R antagonist such as an antagonist that binds to IL-4Rα. The methods also require the administration of an immunogenic component, such as a component comprising one or more antigens. Non-limiting examples of suitable antigens include any one or more of those described in the section above entitled "Antigens". The antigen may be derived from a pathogenic microorganism such as a virus. For example, the methods may comprise administering an antigen from HIV-1 and/or HIV-2, including antigen(s) encoded by any one or more of the gp120 env, gp140 env, gp160 env, p18, gag, pol, vif, vpr, vpu, tat, rev, and nef genes, and combinations thereof (e.g. gag/pol, gag/pol/env).

The methods may involve simultaneous administration of the IL-4 antagonist(s) and immunogenic component(s), or, involve sequential administration of the IL-4 antagonist(s) followed by the immunogenic component(s) (or *vice versa*)*.* When administered simultaneously, the IL-4 antagonist(s) and immunogenic component(s) may be provided in the same composition, or in separate compositions. The compositions may comprise one or more nucleic acid constructs or recombinant viruses comprising polynucleotide sequence(s) encoding the IL-4 antagonist(s) and/or immunogenic component(s) (e.g. one or more recombinant poxviruses such as a recombinant vaccinia virus, recombinant fowlpox virus, and/or a recombinant MVA virus). Non-limiting examples of suitable nucleic acid constructs and recombinant viruses are set out above in the subsections entitled "nucleic acid constructs", "recombinant viruses" and "exemplary recombinant viruses".

The present inventors have identified that the magnitude of antigen-specific immune cell responses and/or the avidity of immune cells for an antigen may be further improved by transient antagonism of IL-4/IL-4R signalling in the local milieu of an immune response to the antigen. For example, the generation of high avidity antigen-specific immune cells (e.g. memory CD8⁺ T cells) may be improved by transient expression (e.g. for a period of less than 1, 2, 3, 4, 5, 6 7, 14 or 21 days) of an IL-4 antagonist (e.g. an IL-4Rα antagonist) upon initial administration of the antigen.

Exemplary formulations for administration are described in the subsection above entitled "*formulations and vaccines".* Exemplary dosages and routes of administration are described in the section below entitled *"Dosages and routes of administration* ".

In general, the compositions, medicaments and vaccines are administered to the subject in a therapeutically effective amount. A "therapeutically effective amount" includes within its meaning a non-toxic but sufficient amount of the active component(s) for use in the present invention to provide the desired therapeutic effect. The exact amount required will vary from subject to subject depending on factors such as the species being treated, the age and general condition of the subject, the severity of the condition being treated, the nature of the infection being prevented, the particular agent being administered, the mode of administration, and so forth. Thus, it is not possible to specify an exact "therapeutically effective amount". However, for any given case, an appropriate "therapeutically effective amount" may be determined by one of ordinary skill in the art using only routine experimentation.

"Subjects" as contemplated herein include mammals (e.g. humans) and individuals of any species of social, economic or research importance including, but not limited to, ovine, bovine, equine, porcine, feline, canine, avian, primate, and rodent species.

The methods of the present invention may be used to induce an antigen-specific immune response in a subject against an antigen co-administered with an IL-4 antagonist. The antigen-specific immune response may comprise T lymphocytes (e.g. CD8⁺ T lymphocytes). It will be understood that "inducing" an antigen-specific immune response as contemplated herein includes inciting an immune response as well as modulating a previously existing immune response (e.g. enhancing or existing immune response). Co-administration of the antigen with an IL-4 antagonist may enhance immune responses specific for the antigen above those achieved by administration of the antigen without the IL-4 antagonist.

Additionally or alternatively, the methods of present invention may be used to increase the avidity of immune cells for an antigen co-administered to a subject with an IL-4 antagonist. The immune cell may be a T lymphocyte (e.g. a CD8⁺ T lymphocyte). By increasing the immune cell avidity for the antigen, the quantity or concentration of the antigen required to elicit a response in the immune cell/immune cell population is decreased. Co-administration of the antigen with an IL-4 antagonist may enhance avidity of the immune cell for the antigen above the avidity that would be achieved by administration of the antigen without the IL-4 antagonist.

Additionally or alternatively, the methods of present invention may be used to increase the number of immune cells specific for an antigen in a subject. The immune cell may be a T lymphocyte (e.g. a CD8⁺ T lymphocyte). Co-administration of the antigen with an IL-4 antagonist may increase the number of immune cells specific for the antigen above the number that would be achieved by administration of the antigen without the IL-4 antagonist.

The induction of antigen-specific immune responses and the number and avidity of antigen-specific immune cells can be determined using standard methods known to those skilled in the field. Exemplary methods include, but are not limited to, solid-phase heterogeneous assays (e.g. enzyme-linked immunosorbent assay), solution phase assays (e.g. electrochemiluminescence assay), amplified luminescent proximity homogeneous assays, flow cytometry, intracellular cytokine staining, functional T-cell assays, functional B-cell assays, functional monocyte-macrophage assays, tetramer binding and dissociation assays, pentamer binding and dissociation assays, dendritic and reticular endothelial cell assays, measurement of NK cell responses, oxidative burst assays, cytotoxic-specific cell lysis assays, and phagocytosis and apoptosis evaluation.

The methods may comprise administering a priming dose of the antigen and interleukin-4 receptor antagonist, and subsequently administering a later booster dose of the antigen and interleukin-4 receptor antagonist. For example, the booster dose may be administered at least 7, 14, 21 or 28 days, at least 1, 2, 3, 4, 5, or 6 months, or at least 1, 2, 3, 4, or 5 years after the priming dose. The antigen and IL-4 antagonist of the priming vaccine dose may be administered separately or sequentially. The antigen and IL-4 antagonist of the booster dose may be administered separately or sequentially. The priming and booster dose may be administered by the same or different routes. For example, the priming and booster doses may both be administered mucosally (e.g. intranasally), intramuscularly, intravenously or subcutaneously. Alternatively, the priming dose may be administered mucosally (e.g. intranasally) to induce mucosal antigen-specific immune cells, and the booster dose administered intramuscularly, subcutaneously or intravenously to induce systemic antigen-specific immune cells.

### Dosages and routes of administration

Compositions, medicaments and vaccines of the present invention can be administered to a recipient by standard routes, including, but not limited to, parenteral (e.g. intravenous, intraspinal, subcutaneous or intramuscular), oral, topical, or mucosal routes (e.g. intranasal).

The compositions, medicaments and vaccines may be administered to a recipient in isolation or in combination with other additional therapeutic agent(s). In embodiments where they are administered with therapeutic agent(s), the administration may be simultaneous or sequential (i.e. composition/medicament/vaccine administration followed by administration of the agent(s) or *vice versa*)*.*

In general, the compositions, medicaments and vaccines may be formulated in a manner compatible with the route of administration and physical characteristics of the recipient (including health status) and in such a way that it elicits the desired effect(s) (i.e. therapeutically effective, immunogenic and/or protective). For example, the appropriate dosage of compositions, medicaments and vaccines of the present invention may depend on a variety of factors including, but not limited to, a subject's physical characteristics (e.g. age, weight, sex), whether the composition, medicament or vaccine is being used as single agent or adjuvant therapy, the type of MHC restriction of the patient, the progression (i.e. pathological state) of a virus infection, and other factors that may be recognized by one skilled in the art. Various general considerations that may be considered when determining an appropriate dosage are described, for example, in Gennaro et al. (Eds), (1990), "Remington's Pharmaceutical Sciences", Mack Publishing Co., Easton, Pennsylvania, USA; and Gilman et al., (Eds), (1990), "Goodman And Gilman's: The Pharmacological Bases of Therapeutics", Pergamon Press.

One skilled in the art would be able, by routine experimentation, to determine an effective, non-toxic amount of an antigen and/or an IL-4 antagonist as described herein to include in a composition, medicament or vaccine of the present invention for the desired therapeutic outcome. In general, a composition, medicament or vaccine of the present invention may be administered to a patient in an amount of from about 50 micrograms to about 5 mg of active component(s). Dosage may be in an amount of from about 50 micrograms to about 500 micrograms of active component(s). Generally, an effective dosage is expected to be in the range of about 0.0001mg to about 1000mg of active component(s) per kg body weight per 24 hours; typically, about 0.001mg to about 750mg per kg body weight per 24 hours; about 0.01mg to about 500mg per kg body weight per 24 hours; about 0.1mg to about 500mg per kg body weight per 24 hours; about 0.1mg to about 250mg per kg body weight per 24 hours; or about 1.0mg to about 250mg per kg body weight per 24 hours. More typically, an effective dose range is expected to be in the range about 1.0mg to about 200mg per kg body weight per 24 hours; about 1.0mg to about 100mg per kg body weight per 24 hours; about 1.0mg to about 50mg per kg body weight per 24 hours; about 1.0mg to about 25mg per kg body weight per 24 hours; about 5.0mg to about 50mg per kg body weight per 24 hours; about 5.0mg to about 20mg per kg body weight per 24 hours; or about 5.0mg to about 15mg per kg body weight per 24 hours.

Typically, in treatment applications, the treatment may be for the duration of the disease state or condition. Further, it will be apparent to one of ordinary skill in the art that the optimal quantity and spacing of individual dosages will be determined by the nature and extent of the disease state or condition being treated, the form, route and site of administration, and the nature of the particular individual being treated. Optimum conditions can be determined using conventional techniques.

In many instances (e.g. preventative applications), it may be desirable to have several or multiple administrations of a composition, medicament or vaccine of the present invention. For example, the composition, medicament or vaccine may be administered 1, 2, 3, 4, 5, 6, 7, 8, 9, 10, or more times. The administrations may be from about one to about twelve week intervals, and in certain embodiments from about one to about four week intervals. Periodic re-administration may be desirable in the case of recurrent exposure to a particular antigen targeted.

It will also be apparent to one of ordinary skill in the art that the optimal course of administration can be ascertained using conventional course of treatment determination tests.

Where two or more entities are administered to a subject "in conjunction", they may be administered in a single composition at the same time, or in separate compositions at the same time, or in separate compositions separated in time.

Certain embodiments of the present invention involve the administration of composition, medicament or vaccines in multiple separate doses. Accordingly, the methods for the prevention (i.e. vaccination) and treatment of infection described herein encompass the administration of multiple separated doses to a subject, for example, over a defined period of time. Accordingly, the methods for the prevention (i.e. vaccination) and treatment of infection disclosed herein include administering a priming dose. The priming dose may be followed by a booster dose. The booster dose may be for the purpose of re-vaccination. In various embodiments, the composition, medicament or vaccine is administered at least once, twice, three times or more.

### Kits

Agent(s) suitable for performing the methods of the present invention, including compositions, medicaments and vaccines, may be provided as component(s) in kits.

Kits of the present invention may comprise components to assist in performing the methods of the present invention such as, for example, administration device(s), buffer(s), and/or diluent(s). The kits may include containers for housing the various components and instructions for using the kit components in the methods of the present invention.

The kits may be fragmented kits or combined kits as defined herein. Fragmented kits comprise reagents are housed in separate containers, and may include small glass containers, plastic containers etc. Such containers may allow the efficient transfer of reagents from one compartment to another compartment whilst avoiding cross-contamination of reagents, and the addition of agents or solutions of each container from one compartment to another in a quantitative fashion. Typically, a kit of the present invention will also include instructions for using the kit components to conduct the appropriate methods.

For example, the kit may comprise a first container and second container. The first container may comprise an IL-4 antagonist (e.g. such as an antagonist that binds to IL-4Rα). The second container may comprise components comprising or encoding an immunogenic agent (e.g. one or more viral antigens). The kits may also comprise one or more other containers, containing for example, one or more devices to assist in administering component(s) of the kit to a subject as required to perform the methods of the invention.

It will be appreciated by persons of ordinary skill in the art that numerous variations and/or modifications can be made to the present invention as disclosed in the specific embodiments without departing from the spirit or scope of the present invention as broadly described. The present embodiments are, therefore, to be considered in all respects as illustrative and not restrictive.

### EXAMPLES

The present invention will now be described with reference to the following specific examples, which should not be construed as in any way limiting on the scope of the invention.

### Example 1: preparation of vaccines

### - Design and preparation of murine IL-4 mutant sequences

Mouse IL-4C118 cDNA was amplified from total spleen RNA using gene specific primers and the One-Step RT-PCR kit (QIAGEN).

Forward primer 5' GGATCCACCATGGGTCTCAACCCCCAGCTA 3' (**SEQ ID NO: 1**) contained a BamHI (GGATCC) restriction site to facilitate cloning and a consensus Kozak translation initiation sequence (CCACC**ATG**G) overlapping the methionine start codon.

Reverse primer 5' GAATTCTAATCCATTTGCATGATGCTC 3' (**SEQ ID NO: 2**) contained the insertion of an in-frame STOP codon (TAG) to prematurely terminate the amino acid sequence after position 118 on the mature secreted protein, which lacks the 20 amino acid signal peptide encoded by the complete gene sequence. Amino acid 118 of the mature secreted protein thus corresponds to codon position 138 of the complete gene sequence (which includes the amino terminal cleavable signal peptide). Insertion of the stop codon creates a deletion of the two terminal amino acids 119 & 120 removing the essential Tyrosine at position 119 required for cell signalling. The reverse primer also contains an EcoRI restriction site to facilitate DNA sub-cloning.

The 431 bp PCR fragment (**SEQ ID NO: 3**) was directly ligated into the U-tailed vector pDrive and transformed into QIAGEN EZ competent cells (QIAGEN). The PCR fragment was subcloned between the BamHI and EcoRI sites of pBluescriptSK+, to add a downstream HindIII site, and then subcloned as a BamHI-HindIII fragment into pAF09 (see Reference 7) in-frame with the fowlpox virus early/late promoter ATG codon contained in the vector. The mouse IL-4C118 BamHI-HindIII fragment was also subcloned into pTK7.5A (see Reference 9) downstream of the vaccinia virus P7.5 early/late promoter.

### - Construction of FPV-HIV and VV-HIV vaccines co-expressing IL-4C118

Recombinant viruses co-expressing the HIV gag/pol(mut) antigen and mouse IL-4C118 were constructed using parent viruses FPV-HIV 086 and VV-HIV 336 (see Reference 1). Recombinant FPV was constructed by infecting chicken embryo skin (CES) cell cultures with FPV-HIV 086 (MOI 0.05) followed by transfection with pAF09-IL-4C118 using Lipofectamine 2000 (Invitrogen, USA). rFPV were selected by passage of viruses on CES cells in the minimal essential media (MEM) containing MX-HAT (2.5 mg/ml mycophenolic acid, 250 mg/ml xanthine, 100 mg/ml hypoxanthine, 0.4 mg/ml aminopterine and 30 mg/ml thymidine) to select for viruses expressing the gpt (xanthine guanine phosphoribosyltransferase) gene. Plaques containing recombinant viruses were identified using an agar overlay (1% agar in MEM) containing X-gal (200 mg/ml) to detect co-expression of the lacZ gene. Blue staining plaques were picked and 3 to 4 plaque purification rounds were performed under selection media. Recombinant viruses were confirmed by PCR for the presence of the IL-4C118 gene and absence for wild-type virus.

Similarly, rVV were constructed by infecting H143B TK-cells with VV-336 (MOI 0.05) and transfection with pTK7.5A- IL-4C118. Recombinant viruses were selected using MEM containing HAT supplement to select for viruses expressing the Herpes Simplex Virus TK gene contained in the vector. Viruses were plaque purified under selection and purity confirmed similar to rFPV.

Recombinant FPV-092 (HIV gagpol and env) and VV337 (HIV env) (see Reference 1) co-expressing mouse IL-4C118 were similarly constructed using the above methods.

A brief summary description of the IL-4C118 recombinant viruses produced is provided below. As noted above, the construction of each is described in Reference 1, and each is commercially available from the CSIRO biological reagents catalogue (see: http://asnet1-mi.act.csiro.au/index.aspx).
(i) FPV086 is a recombinant fowlpox virus expressing HIV-1 subtype B gagpol (with specific mutations) under the control of PE/L (fpv early/late) and inserted in the F6,7,9 locus of FPV-M3 using pKG10a;
(ii) VV336 is a TK negative recombinant vaccinia virus expressing HIV subtype A/E gagpol (with specific mutations) under the control of PE/L (fpv early/late) inserted in the TK locus of VV-WR-L929 using pJmcs;
(iii) FPV092 is a recombinant fowlpox virus expressing HIV-1 subtype AE gagpol (with specific mutations) under the control of PE/L (fpv early/late) and inserted in the F6,7,9 locus of FPV-090 (AE env) using pKG10a;
(iv) VV337 is aTK negative recombinant vaccinia virus expressing HIV subtype A/E env (with specific mutations) under the control of PE/L (fpv early/late) inserted in the TK locus of VV-WR-L929 using pJmcs.

### - Macaque IL4C123 vaccine

The natural macaque IL-4 cDNA sequence was obtained from the GenBank database (see entry under GenBank/NCBI Reference Sequence: NM_001032904 - **SEQ ID NOs: 8-9**). A synthetic DNA sequence was designed (**SEQ ID NO: 4**) so that a stop codon was introduced immediately following amino acid 123 on the mature secreted protein, deleting the carboxyl-terminal sequences from the recombinant mutant protein. By analogy with the mouse and human IL4 proteins the essential Tyrosine at position 124 required for cell signalling was deleted.

The DNA sequence was modified as follows. A unique upstream sequence containing a BamHI site and a Kozak sequence was included to facilitate subcloning and ensure efficient translation of the protein respectively. A HindIII site was also included at the 3'end to facilitate cloning into expression vectors. The DNA sequence was synthesised to order by Genscript USA Inc. The synthetic DNA was subcloned between the BamHI and HindIII sites of pAF09 as described above and recombinant fowlpox viruses isolated using FPV089 encoding the SIV gagpol gene. FPV089 is a recombinant fowlpox virus expressing SIV gagpol under the control of PE/L (fpv early/late) and inserted in the F6,7,9 locus of FPV-M3 using pKG10a. The construction of FPV089 is described in Reference 1, and it is commercially available from the CSIRO biological reagents catalogue (see: http://asnet1-mi.act.csiro.au/index.aspx).

For insertion and expression of the macaque IL-4C123 and SIV gag/pol genes into Modified vaccinia Ankara virus a series of new vectors were developed based on the MVA "del-III" (**Figure 1** **- SEQ ID NO: 10**) and vaccinia "HindIII-F regions" (**Figure 2** **- SEQ ID NO: 11**) using a GFP-blasticidin S deaminase (BSD) fusion gene as a selectable marker gene (see Reference 8) utilising intergenic insertion sites (see **Figures 1** **and** **2**). A synthetic SIV mac239 gag/pol sequence was used that is optimised for expression in macaque monkeys (**Figure 3** **- SEQ ID NO: 12**). The sequence contains a synthetic poxvirus early/late promoter upstream of the genes and an early transcription terminator sequence (T5NT) downstream flanked by restriction endonuclease site to facilitate cloning. The genes are expressed as a fusion protein resulting from a frame-shift during translation in the overlapping region.

It is envisaged and predicted that the Macaque IL4C123 vaccine produced above will induce improved immune responses against SIV in macaques administered the vaccine, compared to, for example, a similar or identical vaccine comprising the SIVgagpol gene without the mutant IL-4 sequence generated. More specifically, it is envisaged and predicted that the vaccine will induce improved antigen-specific T cell avidity and/or an improved magnitude of antigen-specific T cell responses against the SIV gagpol gene product.

### - Design of human IL-4 mutant sequences

Human IL-4 sequences were also designed with a mutation at residue 123, in view of the high levels of sequence homology between the IL-4 gene and protein in macaques and humans.

A non-limiting example of a human IL-4 mRNA sequence is provided in GenBank/NCBI Reference Sequence: NM_000589.2. **SEQ ID NO: 13** provides mRNA and amino acid sequences for the mature secreted form of human IL-4.

**SEQ ID NO: 5** provides a human IL-4 DNA sequence encoding a mutant IL-4 protein (IL-4C123). The human IL-4C123 was designed by incorporating a stop codon (TGA) at codon position 148 so that the protein will be terminated after amino acid position 123 on the mature secreted protein following deletion of the 24 amino acid signal peptide. Amino acid position one on the mature secreted protein corresponds to codon position 25 on the mRNA. This will result in the generation of a mutant IL-4 lacking the essential Tyrosine at position 124 required for cell signalling. It is envisaged and expected that his protein will bind to both the type I and type II IL-4R without signalling preventing activation by endogenous IL-4 and IL-13.

The sequence above has been optimised for enhancing expression in human cells when used in the context of a vaccine (**SEQ ID NO: 6**). Codon optimisation resulted in the generation of a rare FseI restriction site (GGCCGGCC). This restriction site is used during the sub-cloning process into the fowlpox and MVA vectors so was removed from the proposed optimised human IL4C123 sequence without changing the amino acid sequence

### (SEQ ID NO: 7).

It is envisaged and predicted that the human IL-4 protein mutants encoded by these sequences will be IL-4 receptor antagonists capable of binding to the IL-4 receptor without initiating IL-4 receptor-mediated cell signalling to any significant extent.

Moreover, it is envisaged and predicted that a human vaccine comprising any one or more of the human IL-4 protein sequences in combination with an antigen/antigen-encoding sequence will induce improved immune responses against the antigen in a human administered the vaccine, compared to, for example, a human administered a similar or identical vaccine comprising the antigen/antigen-encoding without the human IL-4 protein mutant(s). More specifically, it is envisaged and predicted that such a human vaccine will induce improved antigen-specific T cell avidity and/or an improved magnitude of antigen-specific T cell responses in a human to which it is administered.

### Example 2: IL-4 antagonist vaccination induces high avidity CD8⁺ T cells

### (i) Materials and methods

- *Immunization of mice:* Pathogen free 6-7 week old female BALB/c (H-2^{d}) mice were obtained from the Animal Breeding Establishment, John Curtin School of Medical Research (JCSMR). All animals were maintained and used in accordance with the Australian National University animal experimentation ethics guidelines. Mice were prime-boost immunized with 1 x 10⁷ pfu rFPV followed by 1 x 10⁷ pfu rVV expressing HIV-1 antigens or/and IL-4R antagonist as described in **Table 1** under mild methoxyfluorane anesthesia two weeks apart using i.n./i.m. - combined mucosal systemic route of vaccination. To evaluate protective immunity at 6 weeks following booster vaccination mice were challenged intranasally with 75 plaque forming units (PFU) of influenza virus PR8 (A/Puerto Rico/8/1934(H1N1)) expressing the K^{d}Gag₁₉₇₋₂₀₅ epitope of HIV in the neuraminidase stalk (higher dose compared to previous studies) as described in Ranasinghe *et al* 2011 (see Reference 2). This was constructed using reverse genetic technology (see References 3 and 4). Body weight was monitored for 10 days after challenge.

**Table 1. Prime boost vaccine strategies used in this study.**

| | **Prime** | **Boost** |
|---|---|---|
| 1 | i.n. FPV-HIV | i.m. VV-HIV |
| 2 | i.n. FPV-HIV IL-4C118 | i.m. VV-HIV |
| 3 | i.n. FPV-HIV | i.m. VV-HIV IL-4C118 |
| 4 | i.n. FPV-HIVIL-IL-4C118 | i.m. VV-HIV IL-4C118 |

| | | |
|---|---|---|
| All rFPV and rVV constructs encode HIV-1 gag/pol antigens. All the viruses described in Table 1 were constructed using FPV-086 and VV-336 expressing the HIV gag/pol(mut) proteins as described in Reference 1. i.n. = intranasal, i.m. = intramuscular. | | |

- *Tetramer staining and dissociation assays:* Allophycocyanin-conjugated K^{d}Gag₁₉₇-₂₀₅ tetramers were synthesised at the Bio- Molecular Resource Facility at The John Curtin School of Medical Research. 2 - 5 x 10⁶ splenocytes or mucosal lymphocytes were stained with anti-CD8a-FITC antibody (BD PharMigen, San Diego, CA) and APC-conjugated K^{d}Gag₁₉₇-₂₀₅tetramer at room temperature and analysed as described previously (see References 2 and 5).

Similarly, following K^{d}Gag₁₉₇₋₂₀₅ tetramer staining the dissociation assays were performed as described previously (see Reference 2). Plates were configured to assess five time points per sample (0-60 min). 50 µg/ml of anti-H-2K^{d} competitive binding antibody (BD PharMigen, San Diego, USA) was added to each well to prevent dissociated tetramer from re-binding and plates were incubated at 37°C, 5%CO₂. At each time point, cells were transferred into ice-cold FACS buffer to stop the reaction, washed and resuspended in 100 µl of FACS buffer containing 0.5% paraformaldehyde. 100,000 events were acquired on a FACs Calibur flow cytometer (Becton-Dickinson, San Diego, USA)) and analysed using Cell Quest Pro software.
- *IFN-γ and IL-2 ELISpot assay:* IFN-γ or IL-2 HIV-specific T cell responses were measured by IFN-γ or IL-2 capture ELISpot assay as described previously (see References 2 and 6). Briefly, 2x10⁵ spleen or GN cells were added to 96-well Millipore PVDF plates (Millipore, MA, Ireland) coated with 5 µg/ml of mouse anti- IFN-γ or IL-2 capture antibodies (BD PharMigen, San Diego, CA), and stimulated for 12 h or 22 h respectively for IL-2 or IFN-γ ELISpot, in the presence of H-2K^{d} immuno-dominant CD8+ T cell epitope, Gag₁₉₇₋₂₀₅ - AMQMLKETI (**SEQ ID NO: 14**) (synthesised at the Bio-Molecular Resource Facility at JCSMR). ConA-stimulated cells (Sigma, USA) were used as positive controls and unstimulated cells as negative controls. For both ELISpot assays, all steps were carried out exactly as described previously (see References 2 and 5). Plotted data are expressed as SFU per 10⁶ T cells and represent mean values ± SD. Unstimulated cell counts were subtracted from each stimulated value before plotting the data. All instances the background SFU counts were extremely low.
- *Intracellular cytokine analysis (ICS):* IFN-γ and TNF-α producing HIV-specific CD8 T cells, were analysed as described in Ranasinghe *et al.* (see References 2 and 5). Briefly, 2 x 10⁶ lymphocytes were stimulated with AMQMLKETI (**SEQ ID NO: 14**) peptide at 37°C for 16 h, and further incubated with Brefeldin A (eBioscience, CA, USA) for 4 h. Cells were surface-stained with CD8-APC (BD PharMigen, San Diego, CA) then fixed and permeabilized prior to intracellular staining with IFN-γ-FITC and TNF-α-PE (BD PharMigen, San Diego, CA). Total 100,000 gated events per sample were collected using FACS Calibur flow cytometer (Becton Dickinson, San Diego, CA), and results were analysed using Cell Quest Pro software. Prior to plotting the graphs the unstimulated background values were subtracted from the data.
- *Statistical analysis of data:* SD or SEM was calculated and p-values determined using a two-tailed, two sample equal variance or unequal variance Student's t-Test. The p-values less than 0.05 were considered significant. Unless stated otherwise experiments were repeated a minimum of three times.

### (ii) Results

- *HIV-1 vaccines that co-express IL-4 soluble antagonist can induce high avidity and high magnitude of systemic and mucosal HIV-specific CD8⁺ T cells:* The i.n./i.m. prime-boost immunization strategy was selected for these studies as HIV is a disease of the mucosae and sustained mucosal and systemic immunity are both desirable. The data indicates that prime-boosting with the HIV vaccines that co-expressed IL-4R antagonist (IL-4C118) (FPV-HIV- IL-4C118/ VV-HIV- IL-4C118) induced long lasting high avidity K^{d}Gag₁₉₇₋₂₀₅-specific CTL (**Figure 4**). IL-4-/- mice vaccinated with FPVHIVgag/pol and VVHIVgag/ppol can generate high avidity CD8 T cells in 14 days but fail to generate high avidity memory CD8 T cells by 8 weeks. It is postulated that IL-4 is thus necessary in the cell milieu for normal immune function, whereas the transient inhibition of IL-4R with IL-4C118 is able to induce long lasting high avidity CTL with greater protection (**Figure 9**). The data also indicate that inclusion of IL-4C118 in the prime was advantageous in generating high avidity HIV-specific CD8 T cell subset (**Figure 4**). In contrast, if the receptor was only included in the booster immunization (i.n. FPV-HIV/ VV-HIV-IL-4C118) the T cell avidity was more similar to that of the control i.n. FPV-HIV/ i.m. VV-HIV vaccination, even though elevated magnitude of HIV-specific CD8 T cells was observed (**Figure 5**).
   i.n. FPV-HIV- IL-4C118/ i.m. VV-HIV- IL-4C118 vaccine strategy also induced elevated IFN-γ producing systemic (**Figure 6**) and mucosal (**Figure 7**) K^{d}Gag₁₉₇₋₂₀₅-specific CTL. More interestingly, the genito-rectal (mucosal) CTL producing enhanced IL-2 and IFN-γ (**Figure 7**) is an exciting prospect for a HIV vaccine as inducing IL-2 with vaccines of this nature can be a difficult task (normally multi-functionally, specifically IL-2 production is considered as a hallmark of protective immunity). The IL-4 antagonist vaccine strategy is able to induce not only elevated HIV-specific effector T cell immunity but also memory CD8 T cells both in the systemic and mucosal compartments (**Figure 9**).
- *HIV-1 IL-4 antagonist vaccine strategy can generate robust protective immunity:* At 8 weeks following booster immunization, mice were challenged with 75 pfu of influenza virus expressing the K^{d}Gag₁₉₇₋₂₀₅ immunodominant epitope and body weights were monitored daily for 10 days. Mice maintaining body weight and not succumbing to influenza virus infection were considered as a measure of protective immunity (see Reference 2). Post challenge IL-13-/- mice that received the control i.n FPV-HIV/i.m. VV-HIV immunization did not lose significant body weight as opposed to the wild type BALB/c mice that receiving the same vaccine (**Figure 8B**). Interestingly, mice that received i.n. FPV-HIV-IL-4C118/ i.m. VV-HIV- IL-4C118 vaccines performed very similar to IL-13-/- mice that received the control vaccines or mice tested with a soluble IL-13 inhibitor vaccine (i.n. FPV-HIV-IL-13RΔ10/ i.m. VV-HIV-IL-13RΔ10) (**Figure 8A & 8B**). From day five post challenge, the recovery rate of the above three vaccination groups was significantly higher compared to the wild type BALB/c mice that received the control vaccination p <0.05. The above protective data correlated well with the dissociation rates of CD8⁺ T splenocytes from IL-13-/- mice given i.n. FPV-HIV/ i.m. VV-HIV vaccine or the BALB/c mice that received i.n. FPV-HIV- IL-13RΔ10/ i.m. VV-HIV- IL-13RΔ10 (**Figure 4**) and also the effector/memory mucosal and systemic immune responses observed (measured by IFN-γ and IL-2 production) (**Figures 6, 7** **and** **9**). The unimmunised mice showed up to 20-24% of body weight loss by day seven and showed signs of recovery by day ten post challenge **(****Figure 8B**). At ten days IL-4 antagonist vaccine showed significantly higher IFN-γ responses compared to the control vaccination strategy. Data clearly indicate that this IL-4 antagonist vaccine strategy can generate excellent protective immunity.
- *magnitude and avidity of T cell responses induced by priming and*/*or booster IL-4 antagonist vaccines in mice:* Experiments were conducted to determine the effect of IL-4 antagonist administration on CD8⁺ T cell avidity and magnitude at primary and/or booster vaccine stages.

T-cell responses were measured by tetramer staining. As shown in **Figure 5****,** boosting with an HIV vaccine that co-express the IL-4 antagonist IL-4C118 [(i) FPV-HIV-IL-4C118/(ii) VV-HIV; or (i) FPV-HIV-IL-4C118/(ii) VV-HIV-IL-4C118] induced high avidity CTL. Priming and boosting with an HIV vaccine that co-express the IL-4 antagonist IL-4C118 [(i) FPV-HIV-IL-4C118/(ii) VV-HIV-IL-4C118] induces both high avidity and high magnitude CD8⁺ T cell responses. However, delivering the IL-4 antagonist only in the booster dose [(i) FPV HIV/(ii) VV HIVIL-4C118] induced only a high magnitude of CD8⁺ T cell responses but did not induce high avidity.
- *systemic T cell responses induced by priming and booster IL-4 antagonist vaccines in mice:* Experiments were conducted to determine the level of systemic CD8⁺ T cell immunity induced by priming and boosting with the IL-4 antagonist. As shown in **Figure 6****,** intracellular cytokine staining indicates that IL-4 antagonist (IL-4C118) (grey bar) delivered in the prime and the booster vaccinations can enhance the induction of systemic HIV-specific CD8⁺ IFNγ⁺ T cells.
- *mucosal T cell responses induced by priming and booster IL-4 antagonist vaccines in mice:* ELISPOT assays were conducted to determine the level of mucosal CD8⁺ T cell immunity induced by priming and boosting with the IL-4 antagonist. As shown in **Figure 7****,** the ELISPOT data indicates that IL-4 antagonist (IL-4C118) delivered in the prime and the booster vaccinations can enhance the induction of HIV-specific mucosal CD8⁺ T cells that can express both IFNγ⁺ and interleukin 2 (IL-2).

### Example 3: Evaluation of FPV HIV IL-4 antagonist vaccine

### (i) Materials and methods

- *immunization of mice for lung antigen presenting studies:* Pathogen free 6-7 week old female BALB/c (H-2^{d}) mice were obtained from the Animal Breeding Establishment, John Curtin School of Medical Research (JCSMR). All animals were maintained and used in accordance with the Australian National University (ANU) animal experimentation ethics guidelines. Mice (n= 3-5) were intranasally immunized with 1 x 10⁷ pfu the different recombinant FPV, VV or MVA HIV vaccine constructs (refer to **Examples 1 and 2** above) under mild methoxyfluorane anaesthesia. Immediately prior to delivery the viruses were diluted in phosphate buffered saline (PBS) final volume of 25 µl/mouse and sonicated 20-30s to obtain an homogeneous viral suspension.

The FPV early/late promoter and HIV gagpol was amplified from FPV092 using the primers and PCR
FseIFPVel GGGCCGGCCCATTTAGTATCCTAAAATTGAATTG
SalIKG10a CCTTTTTAACAACCATGGGTCGAC

The product was ligated into the MVA vector along with the GFP-BSD cassette selection was used to construct the pMVA "F-site" described in **Figure 2****.** Recombinant MVA-HIV gagpol was selected by Blasicidine resistance on chick skin cells and confirmed by GFP expression and PCR for gag.
- *preparation of lung cell samples:* Lung samples were first cut into small pieces and digested in 2 mL of complete RPMI buffer containing 2 mg/mL collagenase (Sigma, USA), 2.4 mg/mL dispase (GIBCO,USA), and 5 Units/mL DNAse (Calbiochem, USA) at 37°C for 1 h with gentle vortexing. Sample was then filtered through a cell strainer, rinsed with complete RPMI, red cells lysed and filtered through sterile gauze to remove debris as described previously (see References 2, 10 and 11). The single cell suspensions was then kept at 4°C for minimum of 4-6 h for recovery prior to performing assays since we have observed that digestion can down regulate expression of some surface markers.
- *evaluation of antigen presenting cells using FACS:* BALB/c and IL-13 KO mice were immunised with control FPV-HIV and the other group of BALB/c mice were immunised with the different vaccines. 24 hours post-vaccination, mice were euthanized and lungs were collected and single cell suspensions were prepared as described above. From each sample 4 x 10⁶ cells were aliquoted, and firstly cells were incubated with Fc block antibody (anti-mouse CD16/CD32 Fc Block, BD Biosciences, USA), for 20 min at 40°C and cells were surface stained for 30 min at 40°C with fluorescent labelled anti MHCII I-A^{d}, CD11c, CD11b, B220, CD8a, CD103 (Biolegend, USA or e-Biosciences, USA), antibodies. Cells were fixed and total 500,000 gated events per sample were collected using Fortessa flow cytometer (Becton Dickinson, San Diego, CA), and results were analysed using Cell Quest Pro software.
- *Immunization of mice for antibody studies:* Pathogen free 6-7 week old female BALB/c (H-2^{d}) mice were obtained from the Animal Breeding Establishment, John Curtin School of Medical Research (JCSMR). All animals were maintained and used in accordance with the Australian National University (ANU) animal experimentation ethics guidelines. Mice (n = 5) were prime-boost immunized with 1 x 10⁷ pfu rFPV followed by 1 x 10⁷ pfu rVV expressing HIV-1 antigens or/and IL-4 antagonist under mild methoxyfluorane anaesthesia two weeks apart using i.n./i.m. - combined mucosal systemic route of vaccination. Immediately prior to delivery rFPV and rVV were diluted in phosphate buffered saline (PBS) and sonicated 20-30s to obtain an homogeneous viral suspension, intranasal rFPV was given in a final volume of 20 - 25 µl and i.m. rFPV or rVV were delivered, 50 µl per quadriceps.
- *Serum collection:* Serum was collected from pre-immune mice, and also 2-8 weeks post booster immunisation. Blood was collected by tail vein puncture and serum was separated by centrifugation and stored at -20°C until assayed.

*HIV-1 p24 Gag-specific serum Enzyme-linked Immunosorbent assay (ELISA):* ELISA was used to determine HIV-1 p24 Gag-specific IgG1 and IgG2a serum antibody titres. Falcon Microtest III plates (Becton Dickinson, Oxnard, CA) were coated with HIV-1 p24 Gag (kindly supplied by the NIH AIDS Research and Reference Reagent Program) in borate buffer (Pierce) overnight at 4°C. Plates were washed 5 times with 0.05% Tween20 in PBS (PBST), and non-specific binding sites were blocked by adding 5% skim milk/PBST, at 200 µl/well for 2 hours at 37°C. Plates were then washed as before, and serum samples diluted in 5% skim milk/PBST were added in a volume of 50 µl to each well. Serum samples were diluted two-fold from 1/50 to 1/400. Plates were incubated for 1.5 h at 37°C and washed as indicated with PBST. Secondary antibody, biotin-conjugated anti-mouse IgG1 or anti-mouse IgG2a (Southern Biotechnology Associates, Birmingham, AL) diluted to 1:500 in 1% bovine serum albumin/ PBST (Sigma) (BSA/PBST) was added to respective wells in a 50 µl volume, and incubated overnight at 4°C. Plates were washed 5 times with PBST, and 50 µl of horseradish peroxidase-conjugated streptavidin (HRP-SA, Amersham Life Science) diluted 1:1000 in 1% BSA/PBST was added to each well. Plates were incubated at 37°C for 1.5 h, washed 5 times with PBS and antibodies were detected using 0.01 mg/ml Tetramethyl-benzidine (TMB) (Sigma) substrate dissolved in dimethyl sulfoxide (Sigma) and diluted in TMB citrate/phosphate substrate buffer (Sigma). Optical densities (OD) in each well were read at 405 nm at 15 and 30 min.

### (ii) Results

To assess how the intranasal IL-4 antagonist prime induces high avidity CD8 T cells following i.n. delivery, APC subsets in the lung mucosae were evaluated. BALB/c and IL-13-/- mice (n = 3) were intranasally immunized as described above with the different vaccines. 24 hours post-immunisation lung harvested and cells were stained as described above.

**Figure 10** provides a series of dotplots setting out the antigen presenting cell (APC) subset gating strategy used for evaluating APC subsets in lung mucosae. The FACS plots display cells pre-gated on live cells followed by doublet discrimination based on forward scatter (FSC) and side scatter (SSC), and gated on MHCII I-A^{d}+ and CD11c+ population, the top panel (R5 gate) Then using this MHCII I-A^{d}+CD11c+ gate lung APC subsets expressing various levels of CD11b, B220, CD103, CD8 were analysed.

CD103 associated antigen presenting cell subsets of lung mucosae from mice were evaluated 24 hours post priming vaccination. As shown in **Figure 11** intranasal (i.n.) FPV-HIV-C118 delivery induced an elevated IA^{d}+ CD11c+ CD11b+ CD103- APC subset and a reduced IA^{d}+ CD11c+ CD11b- CD103+ population in the lung mucosae compared to wild type BALB/c mice or the IL-13 -/- (knock out) mice receiving the control vaccine. The data suggests that the IA^{d}+ CD11c+ CD11b+ CD103- population plays an important role in inducing high avidity CTL. In general i.n. FPV prime induces an APC subset that expresses elevated CD11b and lower CD103 in the lung mucosae compared to rVV or rMVA vectors (upper panels).

B220 associated antigen presenting cell subsets were evaluated in lung mucosae 24 hours post-priming vaccination (**Figure 12**). Intranasal FPV-HIV-C118 delivery induced IA^{d}+ CD11c+ CD11b+ B220- (see upper left population in far right panel of central row) antigen presenting cell subset in the lung mucosae compared to wild type BALB/c (top left panel) or IL-13-/- mice (central row, second panel from left) given the FPV-HIV control vaccine. Similarly, FPV-HIV-C118 delivery induced elevated APC subset that was IA^{d}+ CD11c+ CD11b-CD8-. The intranasal recombinant FPV vectors induced lower IA^{d}+ CD11c+ CD11b- B220+ compared to VV-HIV or MVA-HIV (see lower right population in far right panel of central row).

The top right arrow (a) in **Figure 12** highlights the importance of intranasal FPV priming compared to VV or MVA priming. The central right arrow (b) in **Figure 12** shows that intranasal delivery of the IL-4R antagonist adjuvanted FPV-HIV vaccine induces an excellent CD11+B220- dendritic cell subset in lung mucosae, and that the FPV-HIV-C118 prime induces the highest IA^{d}+ CD11c+ CD11b+B200- dendritic cell percentage. The bottom right arrow in **Figure 12**(c) shows that intranasal delivery of VV enhanced the CD11b- B200+ DC subset in the lung mucosae. The clearly demonstrates that intanasal FPV priming is beneficial to inducting high avidity CD8 T cells which are induced during the priming vaccination rather than the booster.

CD8-associated antigen presenting cell subsets in the lung mucosae were also evaluated 24 hours post priming vaccination (**Figure 13**). Intranasal FPV-HIV-C118 delivery induced a lower percentage of dendritic cells that were IA^{d}+ CD11c+ CD11b-CD8+ (lower right population in bottom right panel - arrow indicates CD11b-CD8+ cells) in the lung mucosae compared to wild type BALB/c (top left panel) or IL-13-/- mice (bottom left panel) given the FPV-HIV control vaccine. Similarly, FPV-HIV-C118 delivery induced elevated APC subset that was IA^{d}+ CD11c+ CD11b+CD8- (bottom right panel - upper left population). Intransal recombinant FPV-HIV, VV-HIV or MVA-HIV vaccination induced similar percentages of DC that were IA^{d}+ CD11c+ CD11b- CD8+, suggesting that the vector used did not influence this DC subset.

**Figure 14** demonstrates enhanced serum IgG1 response to p24Gag following HIV IL-4R antagonist adjuvanted vaccination (2 weeks post booster vaccination). These responses are expected to increase over time as optimum antibody responses are onserved 4-8 weeks post vaccination. The responses obesreved were much greater than IL-13 KO mice given the control vaccine or the group that received the IL-13Ra2 adjuvanted vaccine (Reference 11).

### (iii) Discussion

These results and additional observations highlight at least the following points:
A. The novel FPV HIV IL-4 antagonist vaccine when delivered intranasally (i.n.) can recruit unique antigen presenting cell (APC) subsets to the lung mucosae 12-24h post vaccination which is most likely responsible for the induction of high avidity CTL. Data suggest that APC subset IAd⁺CD11c⁺CD11b⁺CD103⁻B220⁻CD8⁻ induced following FPV-HIV-IL-4 antagonist vaccines plays a critical role in modulating T cell avidity (Figures 10-13). These vaccines behave very similar to IL-13-/- mice given the control FPV-HIV vaccine (Figures 11-13). Current APC data clearly indicate that IL-4 and IL-13 depleted cell milieu can chemoattract unique APC subsets into the lung mucosae, promoting the induction of high avidity CD8 T cells;
B. intranasal rFPV prime can recruit different APC subsets to the lung mucosae compared to recombinant vaccinia or modified vaccinia ankara (MVA) suggesting that the priming vector also plays an important role in modulating avidity (fig 1-3). Specifically, IAd⁺CD11c⁺CD11b⁻CD103+ population induced following VV and MVA appears to have a negative implication on avidity;
C. rFPV is an excellent safe intranasal delivery vector and it does not cross the blood-brain barrier in mice (data not shown).
D. intranasal FPV HIV IL-4 antagonist (C118) prime/ i.m. VV HIV C118 booster vaccination strategy can also induce elevated p24 gag IgG1 antibody responses even at 2 week post booster vaccination. These responses will normally enhance further 4-8 weeks post booster vaccination.

### References for Examples 1, 2 and 3

1. Coupar, B.E.H., et al. Fowlpox virus vaccines for HIV and SIV clinical and pre-clinical trials. Vaccine 24, 1378-1388 (2006).
2. Ranasinghe, C., et al. A comparative analysis of HIV-specific mucosal/systemic T cell immunity and avidity following rDNA/rFPV and poxvirus-poxvirus prime boost immunisations. Vaccine 29, 3008-3020 (2011).
3. Cukalac, T., et al. Narrowed TCR diversity for immunised mice challenged with recombinant influenza A-HIV Env(311-320) virus. Vaccine. 27, 6755-6761 Epub 2009 Sep 6758 (2009).
4. Sexton, A., et al. Evaluation of recombinant influenza virus-simian immunodeficiency virus vaccines in macaques. J Virol. 83, 7619-7628 Epub 2009 May 7613 (2009).
5. Ranasinghe, C., et al. Evaluation of fowlpox-vaccinia virus prime-boost vaccine strategies for high-level mucosal and systemic Iimmunity against HIV-1. Vaccine 24, 5881-5895 (2006).
6. Ranasinghe, C., et al. Mucosal HIV-1 pox virus prime-boost immunization Induces high-avidity CD8+ T cells with regime-dependent cytokine/granzyme B profiles. J Immunol. 178, 2370-2379 (2007).
7. Heine, G & Boyle, D. Infectious bursal disease virus structural protein VP 2 expressed by a fowlpox virus recombinant confers protection against disease in chickens. Archives of Virology. 131, 277-292 (1993).
8. Wong, YC., et al. Engineering recombinant poxviruses using a compact GFP-blasticidin resistance fusion gene for selection. Journal of Virological Methods. 171, 295-298 (2011).
9. Coupar B.E.H., et al. A general method for the construction of recombinant vaccinia viruses expressing multiple foreign genes. Gene 68:1-10 (1988).
10. Xi Y, Day SL, Jackson RJ, Ranasinghe C. Role of novel type I interferon epsilon in viral infection and mucosal immunity. Mucosal Immunol 2012 May23;5(6):610--22
11. Ranasinghe C, Trivedi S, Stambas J, Jackson RJ. Unique IL-13Rα2 based HIV-1 vaccine strategy to enhance mucosal immunity, CD8+ T cell avidity and protective immunity. Mucosal Immunol 2013; 13th Feb Advance online.

### Embodiments of the invention will now be described in the following numbered paragraphs:

1. A method for inducing an antigen-specific immune response in a subject, the method comprising administering the antigen to the subject in combination with an interleukin-4 receptor (IL-4R) antagonist.
2. A method for increasing the avidity of immune cells for an antigen in a subject, the method comprising administering the antigen to the subject in combination with an interleukin-4 receptor (IL-4R) antagonist.
3. A method for increasing the number of immune cells specific for an antigen in a subject, the method comprising administering the antigen to the subject in combination with an interleukin-4 receptor (IL-4R) antagonist.
4. The method according to paragraph 2 or paragraph 3, wherein the immune cell is a T-lymphocyte.
5. The method according to any one of paragraphs 2 to 4, wherein the immune cell is a CD8⁺ T-lymphocyte.
6. The method according to any one of paragraphs 1 to 5, wherein the antigen is a viral antigen.
7. The method according to any one of paragraphs 1 to 6, wherein the antigen is a human immunodeficiency virus antigen.
8. The method according to any one of paragraphs 1 to 7, wherein the antigen is a human immunodeficiency virus antigen selected from a gag, pol, or env gene product, or any combination thereof.
9. The method according to any one of paragraphs 1 to 8, wherein the antigen and interleukin-4 receptor antagonist are administered to the subject sequentially.
10. The method according to any one of paragraphs 1 to 8, wherein the antigen and interleukin-4 receptor antagonist are administered to the subject simultaneously.
11. The method according to any one of paragraphs 1 to 10, wherein the antigen and interleukin-4 receptor antagonist are administered to the subject as a priming dose.
12. The method according to any one of paragraphs 1 to 11, wherein said administering comprises administering a polynucleotide encoding the antigen, a polynucleotide encoding the interleukin-4 receptor antagonist, or both.
13. The method according to paragraph 12, wherein any one or more of said polynucleotides is a component of a recombinant virus.
14. The method according to any one of paragraphs 1 to 13, wherein the IL-4R antagonist is an interleukin-4 receptor alpha chain (IL-4Rα) antagonist capable of binding to IL-4Rα and preventing IL-4R signalling.
15. The method according to any one of paragraphs 1 to 14, wherein the IL-4R antagonist is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.
16. The method according to any one of paragraphs 1 to 15, comprising administering a booster dose to the subject, wherein the booster dose comprises the antigen and an IL-4R antagonist.
17. The method according to paragraph 16, wherein the antigen and IL-4R antagonist of the booster dose are administered simultaneously.
18. The method according to paragraph 16, wherein the antigen and IL-4R antagonist of the booster dose are administered sequentially.
19. The method according to any one of paragraphs 16 to 18, wherein administering the booster dose comprises administering a polynucleotide encoding the antigen, a polynucleotide encoding the IL-4R antagonist, or both.
20. The method according to paragraph 19, wherein any one or more of said polynucleotides is a component of a recombinant virus.
21. The method according to any one of paragraphs 16 to 20, wherein the IL-4R antagonist is an interleukin-4 receptor alpha chain (IL-4Rα) antagonist capable of binding to IL-4Rα and preventing IL-4R signalling.
22. The method according to any one of paragraphs 16 to 21, wherein the IL-4R antagonist of the booster dose is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.
23. The method according to paragraph 15 or paragraph 22, wherein the mutation is selected from: R121D, Y124D, a deletion of a fragment encoded by exon 2 of an interleukin-4 gene, or any combination thereof.
24. A composition comprising:
   (i) an antigen and/or a polynucleotide encoding the antigen; and
   (ii) an interleukin-4 receptor (IL-4R) antagonist and/or a polynucleotide encoding the IL-4R antagonist.
25. The composition according to paragraph 24, wherein any one or more of said polynucleotides is a component of a recombinant virus.
26. The composition according to paragraph 24 or paragraph 25, wherein the antigen is a viral antigen.
27. The composition according to any one of paragraphs 24 to 26, wherein the antigen is a human immunodeficiency virus antigen selected from a gag, pol, or env gene product, or any combination thereof.
28. The composition according to any one of paragraphs 24 to 27, wherein the IL-4R antagonist is an interleukin-4 receptor alpha chain (IL-4Rα) antagonist capable of binding to IL-4Rα and preventing IL-4R signalling.
29. The composition according to any one of paragraphs 24 to 28, wherein the IL-4R antagonist is a human interleukin-4 molecule lacking a signal peptide and comprising one or more mutations selected from: a mutation at residue 121, a mutation at residue 124, a deletion of one or more residues after position 123, a deletion of a fragment encoded by exon 2 of an interleukin-4 gene, or any combination thereof.
30. The composition according to paragraph 29, wherein the mutation is selected from: R121D, Y124D, a deletion of a fragment encoded by exon 2 of an interleukin-4 gene, or any combination thereof.
31. The composition according to any one of paragraphs 24 to 30, wherein any one or more of said polynucleotides is a component of a recombinant virus.
32. The composition according to paragraph 31, wherein the recombinant virus is selected from the group consisting of a poxvirus, a vaccinia virus, an attenuated vaccinia virus (e.g. NYVAC), a fowlpox virus, a canarypox virus, and a modified vaccinia ankara (MVA) virus.
33. A method for preventing or treating an infection in a subject, the method comprising administering to the subject the composition according to any one of paragraphs 24 to 32.
34. A method of vaccinating a subject against an infection, the method comprising administering a primer dose and a booster dose of the composition according to any one of paragraphs 24 to 30 to the subject.
35. The method according to paragraph 33 or paragraph 34, wherein the infection is human immunodeficiency virus infection and the composition comprises a human immunodeficiency virus antigen selected from a gag, pol, or env gene product, or any combination thereof.
36. The method according to paragraph 13 or paragraph 20, wherein the recombinant virus is selected from the group consisting of a poxvirus, a vaccinia virus, an attenuated vaccinia virus (e.g. NYVAC), a fowlpox virus, a canarypox virus, and a modified vaccinia ankara (MVA) virus.
37. The method according to paragraph 13, 20 or 36, wherein the recombinant virus transiently expresses the interleukin-4 receptor (IL-4R) antagonist in the subject.

It should be understood that various changes and modifications to the presently preferred embodiments described herein will be apparent to those skilled in the art. Such changes and modifications can be made without departing from the spirit and scope of the present invention and without diminishing its intended advantages. It is therefore intended that such changes and modifications be covered by the following claims:

SEQUENCE LISTING

<110>  THE AUSTRALIAN NATIONAL UNIVERSITY

<120>  Vaccination with Interleukin-4 Antagonists

<130>  SPRBE/P57261EPdiv1

<150>  EP 13800233.2
<151>  2013-06-05

<150>  PCT/AU2013/000589
<151>  2013-06-05

<150>  AU 2012902345
<151>  2012-06-05

<160>  17

<170>  PatentIn version 3.5

<210>  1
<211>  30
<212>  DNA
<213>  Mus musculus

<400>  1
ggatccacca tgggtctcaa cccccagcta                                        30


<210>  2
<211>  27
<212>  DNA
<213>  Mus musculus

<400>  2
gaattctaat ccatttgcat gatgctc                                           27


<210>  3
<211>  431
<212>  DNA
<213>  Mus musculus

<400>  3
ggatccacca tgggtctcaa cccccagcta gttgtcatcc tgctcttctt tctcgaatgt      60

accaggagcc atatccacgg atgcgacaaa aatcacttga gagagatcat cggcattttg     120

aacgaggtca caggagaagg gacgccatgc acggagatgg atgtgccaaa cgtcctcaca     180

gcaacgaaga acaccacaga gagtgagctc gtctgtaggg cttccaaggt gcttcgcata     240

ttttatttaa aacatgggaa aactccatgc ttgaagaaga actctagtgt tctcatggag     300

ctgcagagac tctttcgggc ttttcgatgc ctggattcat cgataagctg caccatgaat     360

gagtccaagt ccacatcact gaaagacttc ctggaaagcc taaagagcat catgcaaatg     420

gattagaatt c                                                          431


<210>  4

<211>  461
<212>  DNA
<213>  Macaca mulatta

<400>  4
ggatccgcca ccatgggtct cacctcccaa ctgcttcccc ctctgttctt cctgctagca      60

tgtgccggca actttgccca cggacacaac tgccatatcg ccttacggga gatcatcgaa     120

actctgaaca gcctcacaga gcagaagact ctgtgcacca agttgaccat aacggacatc     180

cttgctgcct ccaagaacac aactgagaag gaaaccttct gcagggctgc gactgtgctc     240

cggcagttct acagccacca tgagaaggac actcgctgcc tgggtgcgac tgcacagcag     300

tttcacaggc acaagcagct gatccgattc ctgaaacggc tcgacaggaa cctctggggc     360

ctggcgggct tgaactcctg tcctgtgaag gaagccagcc agagtacgtt ggaagacttc     420

ttggaaaggc taaagacgat catgaaagag aaatgaagct t                         461


<210>  5
<211>  444
<212>  DNA
<213>  Homo sapiens

<400>  5
atgggtctca cctcccaact gcttccccct ctgttcttcc tgctagcatg tgccggcaac      60

tttgtccacg gacacaagtg cgatatcacc ttacaggaga tcatcaaaac tttgaacagc     120

ctcacagagc agaagactct gtgcaccgag ttgaccgtaa cagacatctt tgctgcctcc     180

aagaacacaa ctgagaagga aaccttctgc agggctgcga ctgtgctccg gcagttctac     240

agccaccatg agaaggacac tcgctgcctg ggtgcgactg cacagcagtt ccacaggcac     300

aagcagctga tccgattcct gaaacggctc gacaggaacc tctggggcct ggcgggcttg     360

aattcctgtc ctgtgaagga agccaaccag agtacgttgg aaaacttctt ggaaaggcta     420

aagacgatca tgagagagaa atga                                            444


<210>  6
<211>  444
<212>  DNA
<213>  Homo sapiens

<400>  6
atgggcctga ccagccagct gctgcccccc ctgttcttcc tgctggcctg cgccggcaac      60

ttcgtgcacg gccacaagtg cgacatcacc ctgcaggaga tcatcaagac cctgaacagc     120

ctgaccgagc agaagaccct gtgcaccgag ctgaccgtga ccgacatctt cgccgccagc     180

aagaacacca ccgagaagga gaccttctgc agagccgcca ccgtgctgag acagttctac     240

agccaccacg agaaggacac cagatgcctg ggcgccaccg cccagcagtt ccacagacac     300

aagcagctga tcagattcct gaagagactg gacagaaacc tgtggggcct ggccggcctg     360

aacagctgcc ccgtgaagga ggccaaccag agcaccctgg agaacttcct ggagagactg 420

aagaccatca tgagagagaa gtga 444

<210> 7
<211> 444
<212> DNA
<213> Homo sapiens

<400> 7
atgggcctga ccagccagct gctgcccccc ctgttcttcc tgctggcctg cgccggcaac 60

ttcgtgcacg gccacaagtg cgacatcacc ctgcaggaga tcatcaagac cctgaacagc 120

ctgaccgagc agaagaccct gtgcaccgag ctgaccgtga ccgacatctt cgccgccagc 180

aagaacacca ccgagaagga gaccttctgc agagccgcca ccgtgctgag acagttctac 240

agccaccacg agaaggacac cagatgcctg ggcgccaccg cccagcagtt ccacagacac 300

aagcagctga tcagattcct gaagagactg gacagaaacc tgtggggctt agctggttta 360

aacagctgcc ccgtgaagga ggccaaccag agcaccctgg agaacttcct ggagagactg 420

aagaccatca tgagagagaa gtga 444

<210> 8
<211> 462
<212> DNA
<213> Macaca mulatta

<400> 8
atgggtctca cctcccaact gcttccccct ctgttcttcc tgctagcatg tgccggcaac 60

tttgcccacg gacacaactg ccatatcgcc ttacgggaga tcatcgaaac tctgaacagc 120

ctcacagagc agaagactct gtgcaccaag ttgaccataa cggacatcct tgctgcctcc 180

aagaacacaa ctgagaagga aaccttctgc agggctgcga ctgtgctccg gcagttctac 240

agccaccatg agaaggacac tcgctgcctg ggtgcgactg cacagcagtt tcacaggcac 300

aagcagctga tccgattcct gaaacggctc gacaggaacc tctggggcct ggcgggcttg 360

aactcctgtc ctgtgaagga agccagccag agtacgttgg aagacttctt ggaaaggcta 420

aagacgatca tgaaagagaa atattcaaag tgtcggagct ga 462

<210> 9
<211> 153
<212> PRT
<213> Macaca mulatta

<400> 9

Met Gly Leu Thr Ser Gln Leu Leu Pro Pro Leu Phe Phe Leu Leu Ala
1               5                   10                  15

Cys Ala Gly Asn Phe Ala His Gly His Asn Cys His Ile Ala Leu Arg

20                  25                  30

Glu Ile Ile Glu Thr Leu Asn Ser Leu Thr Glu Gln Lys Thr Leu Cys
        35                  40                  45

Thr Lys Leu Thr Ile Thr Asp Ile Leu Ala Ala Ser Lys Asn Thr Thr
    50                  55                  60

Glu Lys Glu Thr Phe Cys Arg Ala Ala Thr Val Leu Arg Gln Phe Tyr
65                  70                  75                  80

Ser His His Glu Lys Asp Thr Arg Cys Leu Gly Ala Thr Ala Gln Gln
                85                  90                  95

Phe His Arg His Lys Gln Leu Ile Arg Phe Leu Lys Arg Leu Asp Arg
            100                 105                 110

Asn Leu Trp Gly Leu Ala Gly Leu Asn Ser Cys Pro Val Lys Glu Ala
        115                 120                 125

Ser Gln Ser Thr Leu Glu Asp Phe Leu Glu Arg Leu Lys Thr Ile Met
    130                 135                 140

Lys Glu Lys Tyr Ser Lys Cys Arg Ser
145                 150

<210>  10
<211>  1308
<212>  DNA
<213>  Artificial sequence

<220>
<223>  Synthetic vector

<400>  10
gcggccgcgg cgcgccacct aaagatgcca ttcctgttat tatatccata ggaaaggata      60

gagatgtttg tgaactatta atctcatctg ataaagcgtg tgcgtgtata gagttaaatt     120

catataaagt agccattctt cccatggatg tttccttttt taccaaagga aatgcatcat     180

tgattattct cctgtttgat ttctctatcg atgcggcacc tctcttaaga agtgtaaccg     240

ataataatgt tattatatct agacaccagc gtctacatga cgagcttccg agttccaatt     300

ggttcaagtt ttacataagt ataaagtccg actattgttc tatattatat atggttgttg     360

atggatctgt gatgcatgca atagctgata atagaactta cgcaaatatt agcaaaaata     420

tattagacaa tactacaatt aacgatgagt gtagatgctg ttattttgaa ccacagatta     480

ggattcttga tagagatgag atgctcaatg gatcatcgtg tgatatgaac agacattgta     540

ttatgatgaa tttacctgat gtaggcgaat ttggatctag tatgttgggg aaatatgaac     600

ctgacatgat taagattgct ctttcggtgg ctggtatttg gaaagtttta taggccggcc 660

gcgatcgcat ttagtatcct aaaattgaat tgtaattatc gataataaat ggacggatcc 720

cgggtcgact gcagaagctt tagttgatag aacaaaatac ataattttgt aaaaataaat 780

cactttttat actaatatga cacgattacc aatacttttg ttactaatat cattagtata 840

cgctacacct tttcctcaga catctaaaaa aataggtgat gatgcaactt tatcatgtaa 900

tcgaaataat acaaatgact acgttgttat gagtgcttgg tataaggagc ccaattccat 960

tattctttta gctgctaaaa gcgacgtctt gtattttgat aattatacca aggataaaat 1020

atcttacgac tctccatacg atgatctagt tacaactatc acaattaaat cattgactgc 1080

tagagatgcc ggtacttatg tatgtgcatt ctttatgaca tcgcctacaa atgacactga 1140

taaagtagat tatgaagaat actccacaga gttgattgta aatacagata gtgaatcgac 1200

tatagacata atactatctg gatctacaca ttcaccggaa actagttctg agaaacctga 1260

ttatatagat aattctaatt gctcgtcggt attcgaaatc gcggccgc 1308

<210> 11
<211> 1256
<212> DNA
<213> Artificial sequence

<220>
<223> Synthetic

<400> 11
gcggccgcgg cgcgcccggt atccaagcta gaaaagaact gtaatgcgta tgcggaggtt 60

aataatatta tagatataca gaaattggat atcggagaat gttcggctcc gcccggtcaa 120

catatgcttt tacagatagt taatacagga tccgcggaag caaattgtgg tttacagaca 180

attgttaagt ccttaaataa aatatacgtt ccacctatta tcgaaaaccg attgccgtat 240

tacgatccgt ggtttctagt gggtgtagca attattctag ttatttttac tgtagctatt 300

tgttctatta gacgaaatct ggctcttaaa tacagatacg gaacgttttt atacgtttaa 360

ttaataaaaa aatttaatta caaggtataa aatagtactc catctacgca atcgcgataa 420

tggagggatc taaacgcaga cacgacagtc ggcgactaca acaagaacag gagcagcctc 480

gtccacgtac accgccatca tatgaagaaa ttgcaaaata tggacactca tttaacgtga 540

aaagatttac gaatgaagaa atgtgtctta agaatgatta tccacgaatt atatcatata 600

atcctccacc aaaatagttt tttttggccg gccgcgatcg caagcttagt atatatatat 660

catcatttca tgatgtatac tactgacata gtttcaatgt gaacttttca ctttcttgcc 720

ggttatgaag aatattttta ttttaatggt cattactaat cgtatattat aattgaaaat 780

ggattagttt aatatgacgc tcgtcatggg atcctgctgt ggtagattct gtgacgctaa 840

gaataagaat aagaaggaag atgtagaaga gggaagagaa ggatgttaca attataagaa 900

ccttaatgat ctggatgaat ccgaagcacg tgtagaattt ggaccattat atatgataaa 960

tgaagaaaaa tcagacataa atacattgga tataaaaaga agatatagac acacgataga 1020

gtctgtatat ttctaaaagt ttttataaaa aatgagtaaa atactcacgt ttgttaaaaa 1080

taagataatt gacttgatta ataatgacca aattaaatat tctagagtta taatgataga 1140

agagtccgat agtcttttac cggttgatga ggtgcatgct aaccacggat ttgactgtgt 1200

ggagatgata gatgaaaata taagcaatga gaatatcgaa cagtataagc ggccgc 1256

<210> 12
<211> 4434
<212> DNA
<213> Artificial sequence

<220>
<223> synthetic SIV mac239 gag/pol

<400> 12
gaattcggcc ggccatttag tatcctaaaa ttgaattgta attatcgata ataaatgggc 60

gtgaggaact ccgtgctgtc cggcaagaag gccgacgagc tggagaagat caggctgagg 120

cccaacggca agaagaagta catgctgaag cacgtggtgt gggccgccaa cgagctggac 180

aggttcggcc tggccgagtc cctgctggag aacaaggagg gctgccagaa gatcctgtcc 240

gtgctggccc ccctggtgcc caccggctcc gagaacctga agtccctgta caacaccgtg 300

tgcgtgatct ggtgcatcca cgccgaggag aaggtgaagc acaccgagga ggccaagcag 360

atcgtgcaga ggcacctggt ggtggagacc ggcaccaccg agaccatgcc caagacctcc 420

aggcccaccg ccccctcctc cggcaggggc ggcaactacc ccgtgcagca gatcggcggc 480

aactacgtgc acctgcccct gtcccccagg accctgaacg cctgggtgaa gctgatcgag 540

gagaagaagt tcggcgccga ggtggtgccc ggcttccagg ccctgtccga gggctgcacc 600

ccctacgaca tcaaccagat gctgaactgc gtgggcgacc accaggccgc catgcagatc 660

atcagggaca tcatcaacga ggaggccgcc gactgggacc tccagcaccc ccagcccgcc 720

ccccagcagg gccagctgag ggagccctcc ggctccgaca tcgccggcac cacctcctcc 780

gtggacgagc agatccagtg gatgtacagg cagcagaacc ccatccccgt gggcaacatc 840

tacaggaggt ggattcagct gggcctgcaa aagtgcgtga ggatgtacaa ccccaccaac 900

atcctggacg tgaagcaggg ccccaaggag cccttccagt cctacgtgga caggttctac 960

aagtccctga gggccgagca gaccgacgcc gccgtgaaga actggatgac ccagaccctg 1020

ctgatccaga acgccaaccc cgactgcaag ctggtgctga agggcctggg cgtgaacccc 1080

accctggagg agatgctgac cgcctgccag ggcgtgggcg gccccggcca gaaggccagg 1140

ctgatggccg aggccctgaa ggaggccctg gcccccgtgc ccatcccctt cgccgccgcc 1200

cagcagaggg gccccaggaa gcccatcaag tgctggaact gcggcaagga gggccactcc 1260

gccaggcaat gcagagcccc aagaagacag ggatgctgga aatgtggaaa aatggaccat 1320

gttatggcca aatgcccaga cagacaggcg ggttttttag gccttggtcc atggggaaag 1380

aagccccgca atttccccat ggctcaagtg catcaggggc tgatgccaac tgctccccca 1440

gaggacccag ctgtggatct gctaaagaac tacatgcagt tgggcaagca gcagagagaa 1500

aagcagagag aaagcagaga gaagccttac aaggaggtga cagaggattt gctgcacctc 1560

aattctctct ttggaggaga ccagtagtga ccgcccacat cgagggccag cccgtggagg 1620

tgctgctgga caccggcgcc gacgactcca tcgtgaccgg catcgagctg ggcccccact 1680

acacccccaa gatcgtgggc ggcatcggcg gcttcatcaa caccaaggag tacaagaacg 1740

tggagatcga ggtgctgggc aagaggatca agggcaccat catgaccggc gacaccccca 1800

tcaacatctt cggcaggaac ctgctgaccg ccctgggcat gtccctgaac ttccccatcg 1860

ccaaggtgga gcccgtgaag gtggccctga agcccggcaa ggacggcccc aagctgaagc 1920

agtggcccct gtccaaggag aagatcgtgg ccctgaggga gatctgcgag aagatggaga 1980

aggacggcca gctggaggag gcccccccca ccaaccccta caacaccccc accttcgcca 2040

tcaagaagaa ggacaagaac aagtggagga tgctgatcga cttcagggag ctgaacaggg 2100

tgacccagga cttcaccgag gtgcagctgg gcatccccca ccccgccggc ctggccaaga 2160

ggaagaggat caccgtgctg gacatcggcg acgcctactt ctccatcccc ctggacgagg 2220

agttcaggca gtacaccgcc ttcaccctgc cctccgtgaa caacgccgag cccggcaaga 2280

ggtacatcta caaggtgctg ccccagggct ggaagggctc ccccgccatc ttccagtaca 2340

ccatgaggca cgtgctggag cccttcagga aggccaaccc cgacgtgacc ctggtgcagt 2400

acatggacga catcctgatc gcctccgaca ggaccgacct ggagcacgac agggtggtgc 2460

tccagtccaa ggagctgctg aactccatcg gcttctccac ccccgaggag aagttccaga 2520

aggacccccc cttccagtgg atgggctacg agctgtggcc caccaagtgg aagctgcaaa 2580

agatcgagct gccccagagg gagacctgga ccgtgaacga catccagaag ctggtgggcg 2640

tgctgaactg ggccgcccag atctaccccg gcatcaagac caagcacctg tgcaggctga 2700

tcaggggcaa gatgaccctg accgaggagg tgcagtggac cgagatggcc gaggccgagt 2760

acgaggagaa caagatcatc ctgtcccagg agcaggaggg ctgctactac caggagggca 2820

agcccctgga ggccaccgtg atcaagtccc aggacaacca gtggtcctac aagatccacc 2880

aggaggacaa gatcctgaag gtgggcaagt tcgccaagat caagaacacc cacaccaacg 2940

gcgtgaggct gctggcccac gtgatccaga agatcggcaa ggaggccatc gtgatctggg 3000

gccaggtgcc caagttccac ctgcccgtgg agaaggacgt gtgggagcag tggtggaccg 3060

actactggca ggtgacctgg attcccgagt gggacttcat ctccaccccc cccctggtga 3120

ggctggtgtt caacctggtg aaggacccca tcgagggcga ggagacctac tacaccgacg 3180

gctcctgcaa caagcagtcc aaggagggca aggccggcta catcaccgac aggggcaagg 3240

acaaggtgaa ggtgctggag cagaccacca accagcaggc cgagctggag gccttcctga 3300

tggccctgac cgactccggc cccaaggcca acatcatcgt ggactcccag tacgtgatgg 3360

gcatcatcac cggctgcccc accgagtccg agtccaggct ggtgaaccag atcatcgagg 3420

agatgatcaa gaagtccgag atctacgtgg cctgggtgcc cgcccacaag ggcatcggcg 3480

gcaaccagga gatcgaccac ctggtgtccc agggcatcag gcaggtgctg ttcctggaga 3540

agatcgagcc cgcccaggag gagcacgaca agtaccactc caacgtgaag gagctggtgt 3600

tcaagttcgg cctgcccagg atcgtggcca ggcagatcgt ggacacctgc gacaagtgcc 3660

accagaaggg cgaggccatc cacggccagg ccaactccga cctgggcacc tggcagatgg 3720

actgcaccca cctggagggc aagatcatca tcgtggccgt gcacgtggcc tccggcttca 3780

tcgaggccga ggtgatcccc caggagaccg gcaggcagac cgccctgttc ctgctgaagc 3840

tggccggcag gtggcccatc acccacctgc acaccgacaa cggcgccaac ttcgcctccc 3900

aggaggtgaa gatggtggcc tggtgggccg gcatcgagca caccttcggc gtgccctaca 3960

acccccagtc ccagggcgtg gtggaggcca tgaaccacca cctgaagaac cagatcgaca 4020

ggatcaggga gcaggccaac tccgtggaga ccatcgtgct gatggccgtg cactgcatga 4080

acttcaagag gaggggcggc atcggcgaca tgacccccgc cgagaggctg atcaacatga 4140

tcaccaccga gcaggagatc cagttccagc agtccaagaa ctccaagttc aagaacttca 4200

gggtgtacta cagggagggc agggaccagc tgtggaaggg ccccggcgag ctgctgtgga 4260

agggcgaggg cgccgtgatc ctgaaggtgg gcaccgacat caaggtggtg cccaggagga 4320

aggccaagat catcaaggac tacggcggcg gcaaggaggt ggactcctcc tcccacatgg 4380

aggacaccgg cgaggccagg gaggtggcct agtttttttt gcgatcgcaa gctt 4434

<210> 13
<211> 462
<212> DNA
<213> Homo sapiens

<400> 13
atgggtctca cctcccaact gcttccccct ctgttcttcc tgctagcatg tgccggcaac 60

tttgtccacg gacacaagtg cgatatcacc ttacaggaga tcatcaaaac tttgaacagc 120

ctcacagagc agaagactct gtgcaccgag ttgaccgtaa cagacatctt tgctgcctcc 180

aagaacacaa ctgagaagga aaccttctgc agggctgcga ctgtgctccg gcagttctac 240

agccaccatg agaaggacac tcgctgcctg ggtgcgactg cacagcagtt ccacaggcac 300

aagcagctga tccgattcct gaaacggctc gacaggaacc tctggggcct ggcgggcttg      360

aattcctgtc ctgtgaagga agccaaccag agtacgttgg aaaacttctt ggaaaggcta      420

aagacgatca tgagagagaa atattcaaag tgttcgagct ga                          462

<210>  14
<211>  9
<212>  PRT
<213>  Artificial sequence

<220>
<223>  Synthetic

<400>  14

Ala Met Gln Met Leu Lys Glu Thr Ile
1               5

<210>  15
<211>  153
<212>  PRT
<213>  Homo sapiens

<400>  15

Met Gly Leu Thr Ser Gln Leu Leu Pro Pro Leu Phe Phe Leu Leu Ala
1               5                   10                  15

Cys Ala Gly Asn Phe Val His Gly His Lys Cys Asp Ile Thr Leu Gln
            20                  25                  30

Glu Ile Ile Lys Thr Leu Asn Ser Leu Thr Glu Gln Lys Thr Leu Cys
        35                  40                  45

Thr Glu Leu Thr Val Thr Asp Ile Phe Ala Ala Ser Lys Asn Thr Thr
    50                  55                  60

Glu Lys Glu Thr Phe Cys Arg Ala Ala Thr Val Leu Arg Gln Phe Tyr
65                  70                  75                  80

His Glu Lys Asp Thr Arg Ser His Cys Leu Gly Ala Thr Ala Gln Gln
                85                  90                  95

Phe His Arg His Lys Gln Leu Ile Arg Phe Leu Lys Arg Leu Asp Arg
            100                 105                 110

Asn Leu Trp Gly Leu Ala Gly Leu Asn Ser Cys Pro Val Lys Glu Ala
        115                 120                 125

Asn Gln Ser Thr Leu Glu Asn Phe Leu Glu Arg Leu Lys Thr Ile Met
    130                 135                 140

Arg Glu Lys Tyr Ser Lys Cys Ser Ser
145                 150

<210>  16
<211>  19
<212>  DNA
<213>  Artificial sequence

<220>
<223>  Synthetic

<400>  16
gggccggccc atttagtatc ctaaaattga attg                                    19

<210>  17
<211>  14
<212>  DNA
<213>  Artificial sequence

<220>
<223>  Synthetic

<400>  17
ccttttaac aaccatgggt cgac                                                14

## Claims

1. A combination of an interleukin-4 receptor (IL-4R) antagonist and an antigen for use in a method of inducing a protective antigen-specific immune response in a subject, the method comprising administering the combination to a subject, wherein:
said administration comprises administering one or more polynucleotides encoding the antigen and the IL-4R antagonist to the subject in a priming dose and a booster dose;
wherein the one or more polynucleotides is or are a component of a recombinant virus;
wherein the recombinant virus of the priming dose and the booster dose transiently express the IL-4R antagonist in the subject;
wherein the IL-4R antagonist is an interleukin 4 (IL-4) protein lacking a tyrosine residue at an amino acid position equivalent to position 124 in human IL-4 and inhibits signaling via IL-4R,
wherein the antigen is from an infectious agent, and
wherein the recombinant virus of the priming dose is an avipoxvirus, and the recombinant virus of the booster dose is MVA virus or NYVAC virus.

2. A composition comprising:
(i) a polynucleotide encoding an antigen; and,
(ii) a polynucleotide encoding interleukin-4 receptor (IL-4R) antagonist;
wherein:
the composition is formulated for administration to a subject in a priming dose and a booster dose;
wherein each polynucleotide is a component of a recombinant virus;
wherein the recombinant virus of the priming dose and the booster dose transiently express the IL-4R antagonist in the subject;
wherein the IL-4R antagonist is an interleukin 4 (IL-4) protein lacking a tyrosine residue at an amino acid position equivalent to position 124 in human IL-4 and inhibits signaling via IL-4R,
wherein the antigen is from an infectious agent, and
wherein the recombinant virus of the priming dose is an avipoxvirus, and the recombinant virus of the booster dose is MVA virus or NYVAC virus.

3. The combination for use according to Claim 1, wherein the antigen-specific immune response comprises T-lymphocytes and/or CD8+ T-lymphocytes.

4. The combination for use according to any one of Claims 1 or 3, or the composition according to Claim 2, wherein the antigen is a viral antigen, optionally wherein the antigen is a human immunodeficiency virus (HIV) antigen.

5. The combination for use according to Claim 4, or the composition according to Claim 4, wherein the antigen is an HIV antigen selected from a *gag, pol,* or *env* gene product, or any combination thereof.

6. The combination for use according to any one of Claims 1 or 3to 5, or the composition according to any one of Claims 2, 4, or 5, wherein IL-4R antagonist, comprising a mutation selected from: a substitution of the tyrosine residue at an amino acid position equivalent to position 124 in human IL-4, or a deletion of the tyrosine at an amino acid position equivalent to position 124 in human IL-4.

7. The combination for use according to Claim 6, or the composition according to Claim 6, wherein the substitution is a tyrosine (Y) to aspartic acid (D) substitution at an amino acid position equivalent to position 124 in human IL-4.

8. A composition according to any one of Claims 2, or 4 to 7 for use in a method of preventing or treating a viral infection in a subject, the method comprising administering to the subject a primer dose and a booster dose of the composition.

9. A composition according to any one of Claims 2, or 4 to 7 for use in a method of vaccinating a subject against a viral infection, the method comprising administering to the subject a primer dose and a booster dose of the composition.

10. The combination for use according to any of Claims 1, or 3 to 7, or the composition for use according to Claim 8 or 9, wherein the IL-4R antagonist and the antigen of the primer dose are administered to the subject simultaneously.

11. The combination for use according to any of Claims 1, 3 to 7, or 10, or the composition for use according any one of Claims 8 to 10, wherein the IL-4R antagonist and the antigen of the booster dose are administered to the subject simultaneously.

12. The combination for use according to any one of Claims 1, 3 to 7, 10, or 11, or the composition for use according any one of Claims 8 to 11, wherein the priming dose is administered to the subject mucosally and the booster dose is administered to the subject intramuscularly, subcutaneously, or intravenously.

13. The combination for use according to any one of Claims 1, 3 to 7, or 10 to 12, or the composition for use according any one of Claims 8 to 12, wherein the recombinant virus of the priming dose is canarypox virus or fowlpox virus.

14. The combination for use according to Claim 13, or the composition for use according to Claim 13, wherein the recombinant virus of the priming dose is fowlpox virus.
